(19) Europäisches Patentamt
European Patent Office
Office européen des brevets

(11) Publication number: 0 550 016 A1

(12) **EUROPEAN PATENT APPLICATION**

(21) Application number: 92121923.4

(22) Date of filing: 23.12.92

(51) Int. Cl.5: C07D 401/04, C07D 215/56, C07D 471/04, A61K 31/47

(30) Priority: 31.12.91 KR 9125884

(43) Date of publication of application: 07.07.93 Bulletin 93/27

(84) Designated Contracting States: AT BE CH DE DK ES FR GB GR IE IT LI LU NL PT SE

(71) Applicant: KOREA RESEARCH INSTITUTE OF CHEMICAL TECHNOLOGY
100, Jang-Dong, Yuseong-Ku
Daejeon, 305-606(KR)

(72) Inventor: Kim, Wan Joo
383-7 Dorhyong-dong, Yuseng-ku
Daejeon 305-606(KR)
Inventor: Park, Myung Hwan
431 Dorhyong-dong, Yuseong-ku
Daejeon 305-606(KR)
Inventor: Ha, Jae Du
100 Jang-dong, Yuseong-ku
Daejeon 305-606(KR)
Inventor: Baik, Kyong Up
39-35 Gajang-dong, Seo-ku
Daejeon 302-181(KR)

(74) Representative: Becker, Heinrich Karl Engelbert, Dr. et al
HOECHST AKTIENGESELLSCHAFT Zentrale Patentabteilung P.O. Box 80 03 20
W-6230 Frankfurt am Main 80 (DE)

(54) **Novel quinolone carboxylic acid derivatives and processes for preparing same.**

(57) The present invention relates to certain novel quinolone compounds of the present invention can be represented by the following formula(I) and novel processes for preparing same.

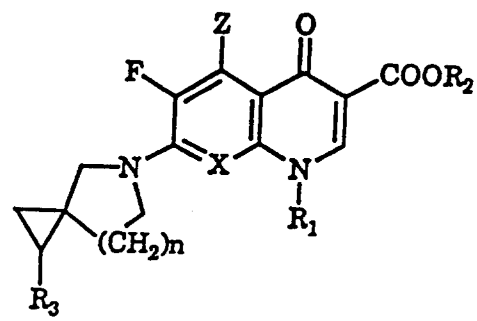

(I)

wherein:

X is a nitrogen atom or a CH, C-halogen or C-methoxy group;

$R_1$ is an optionally substituted $C_{1-7}$ alkyl group, an optionally substituted $C_{3-7}$ cycloalkyl group, an optionally substituted alkenyl group, an optionally substituted aryl group or a divalent group of $-CH_2CH_2{}^*CH(CH_3)-$, $-OCH_2{}^*CH(CH_3)-$or $-SCH_2{}^*CH(CH_3)-$ which forms a ring together with the nitrogen atom to which $R_1$ is attached and with X wherein X is CH;

$R_2$ is a hydrogen atom, a carboxy protecting group or a pharmaceutically acceptable metal or organic cation;

$R_3$ is $-CO_2H$ or $(CH_2)_m$-Y, wherein m is 0 or 1 and Y is a hydroxy group, a $C_{1-4}$ alkoxy group, a $C_{1-4}$

EP 0 550 016 A1

alkoxycarbonyl group(wherein m is 0) or an amino group optionally substituted with one or two $C_{1-4}$ alkyl radicals, with one $C_{1-4}$ alkanoyl radical or with one nitrogen protecting radical metabolizable in vivo, and the carbon atom to which $R_3$ is attached is a chiral carbon atom having the stereochemical configuration of (R), (S) or a mixture thereof;

Z is a hydrogen, chlorine or fluorine atom, a hydroxy group, a methyl group or an optionally substituted amino group; and

n is 1 or 2.

## Field of the Invention

The present invention relates to novel quinolone carboxylic acid derivatives, and pharmaceutically acceptable salts thereof which possess a broad spectrum of potent anti-bacterial activities and are useful as drugs for humans, veterinary drugs or agricultural chemicals and antiseptics; to antibacterial compositions containing one or more of these compounds as active ingredients; and to novel processes for preparing these compounds.

## Description of the Prior Art

Quinolones such as enoxacin, norfloxacin, ofloxacin, ciprofloxacin, tosulfloxacin and the like have been developed and commercially available for some time. However, most of these prior art materials are known to have various side effects for the central nervous system or cardiovascular system; and resistant microorganisms against these drugs, e.g., clinically important methicillin resistant Staphylococcus aureus-(MRSA) have been found to exist.

Therefore, needs have existed for the development of new medicinal compounds which possess a broad scope of potent antibacterial activities and low side effects, and without the problem of resistant microorganisms.

## Summary of the Invention

Unexpectedly, the present inventors have discovered that certain quinolone carboxylic acid derivatives having certain substituents at 7-position of the quinolone nucleus meet the said requirements.

Accordingly, the present invention primarily pertains to said novel quinolone compounds and their pharmaceutically acceptable salts; and novel processes for preparing such compounds.

## Detailed Description of the Invention

The novel quinolone compounds of the present invention can be represented by the following formula-(I):

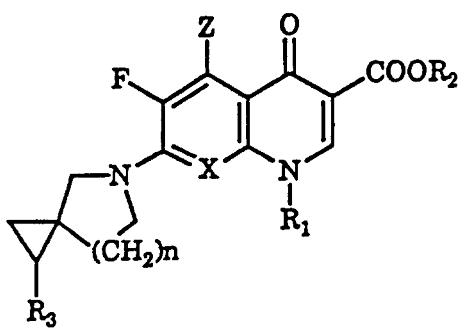

(I)

wherein:

- X is a nitrogen atom or a CH, C-halogen or C-methoxy group;
- $R_1$ is an optionally substituted $C_{1-7}$ alkyl group, an optionally substituted $C_{3-7}$ cycloalkyl group, an optionally substituted alkenyl group, an optionally substituted aryl group or a divalent group of $-CH_2CH_2{}^*CH(CH_3)-$, $-OCH_2{}^*CH(CH_3)-$or $-SCH_2{}^*CH(CH_3)-$ which forms a ring together with the nitrogen atom to which $R_1$ is attached and with X wherein X is C;
- $R_2$ is a hydrogen atom, a carboxy protecting group or a pharmaceutically acceptable metal or organic cation;
- $R_3$ is $-CO_2H$ or $-(CH_2)_m-Y$, wherein m is 0 or 1 and Y is a hydroxy group, a $C_{1-4}$ alkoxy group, a $C_{1-4}$ alkoxycarbonyl group(wherein m is 0) or an amino group optionally substituted with one or two $C_{1-4}$ alkyl radicals, with one $C_{1-4}$ alkanoyl radical or with one nitrogen protecting radical metabolizable in vivo, and the carbon atom to which $R_3$ is attached is a chiral carbon atom having the stereochemical configuration of (R), (S) or a mixture thereof;
- Z is a hydrogen, chlorine or fluorine atom, a hydroxy group, a methyl group or an optionally substituted amino group; and

n is 1 or 2.

With respect to the definition for $R_1$ of the compounds of formula(I), in the case that $R_1$ is an optionally substituted $C_{1-7}$ alkyl group: preferred is a methyl, ethyl, n-propyl, isopropyl, isobutyl, t-butyl or t-pentyl group, or a $C_{1-7}$ alkyl group substituted with at least one halogen atom or hydroxy radical, e.g., 2-fluoroethyl or 2-hydroxyethyl group;
in the case that $R_1$ is an optionally substituted $C_{3-7}$ cycloalkyl group: preferred is a cyclopropyl, cyclobutyl, cyclopentyl, cyclohexyl, 1,2-cis-2-fluorocyclopropyl or 1,2-cis-2-methylcyclopropyl group;
in the case that $R_1$ is an optionally substituted alkenyl group: preferred is a vinyl, isopropenyl or 2-butenyl group;
in the case that $R_1$ is an optionally substituted aryl group: preferred is a phenyl group or a phenyl group substituted with at least one halogen atom, hydroxy, alkyl, alkoxy, amino or nitro radical, e.g., 2-fluorophenyl, 4-fluorophenyl, 2,4-difluorophenyl, 2,5-difluorophenyl, 2-hydroxyphenyl, 4-hydroxyphenyl, 4-methylphenyl, 2,5-dimethylphenyl, 2-methoxyphenyl, 4-methoxyphenyl, 2-fluoro-4-methoxyphenyl, 2-methoxy-4-fluorophenyl, 4-aminophenyl, 2,4-diaminophenyl or 4-nitrophenyl radical; and
in the case that $R_1$ is a $-CH_2CH_2{}^*CH(CH_3)-$, $-OCH_2{}^*CH(CH_3)-$ or $-SCH_2{}^*CH(CH_3)-$ group which forms a ring together with X which is CH and with the nitrogen atom to which $R_1$ is attached, the stereochemical configuration of the chiral carbon(*C) may be (R), (S) or a mixture thereof.

With respect to the definition for $R_2$ of the compounds of formula(I), in the case that $R_2$ is a carboxy protecting group: preferred is an ester moiety which can be readily, hydrolyzed to produce a free carboxylic acid, e.g., a lower alkyl group such as methyl, ethyl, n-propyl, isopropyl, t-butyl, cyclopentyl or cyclohexyl group or an aryl group such as phenyl or benzyl, particularly a group metabolizable in vivo, e.g., a lower alkanoyloxy-lower alkyl group such as acetoxymethyl or pivaloyloxymethyl group, a lower alkoxycarbonyloxy-lower alkyl group such as methoxycarbonyloxy methyl or 1-ethoxycarbonyloxyethyl group, a lower alkoxymethyl group such as methoxymethyl group, a di(lower alkyl)amino-lower alkyl group such as 1-dimethylaminoethyl group, or a 4-methylene-5-methyl-1,3-dioxolene-2-one group; and
in the case that $R_2$ is a pharmaceutically acceptable metal or organic cation: preferred is a cation of alkaline metal or alkaline earth metal such as sodium, potassium, silver, calcium or magnesium cation, an ammonium cation or an organic cation such as a tertiary or quarternary $C_{1-4}$ alkyl ammonium cation.

With respect to the definition for $R_3$ of the compounds of formula(I), in the case that $R_3$ is an amino group substituted with a nitrogen-protecting radical metabolizable in vivo: preferred nitrogen protecting radical is a formyl group, a $C_{1-4}$ alkoxycarbonyl group, a $C_{1-4}$ alkoxycarbonyl methyl group, a 2 or 3-oxoalkyl group, a 4-methylene-5-methyl-1,3-dioxolene-2-one group or a $RCH(NH_2)CO$ group derived from amino acids wherein R is a hydrogen atom, an optionally substituted $C_1$-$C_4$ alkyl group as e.g. methyl, ethyl, propyl, isopropyl, butyl, isobutyl or a phenyl group.

With respect to the definition for Z of the compounds of formula(I), in the case that Z is an optionally substituted amino group: preferred is an amino, acetamino or methylamino group.

With respect to the definition for X of the compounds of formula(I), in the case that X is C-halogen: preferred halogen is a fluorine, chlorine or bromine atom.

Among the compounds of the present invention, preferred are those wherein: $R_1$ is a methyl, ethyl, isopropyl, t-butyl, t-pentyl, 2-fluoroethyl, 2-hydroxyethyl, vinyl, isopropenyl, 2-butenyl, cyclopropyl, 1,2-cis-2-fluorocyclopropyl, cyclobutyl, phenyl, 4-fluorophenyl or 2,4-difluorophenyl group; $R_2$ is a hydrogen atom, a methyl, ethyl, n-propyl, t-butyl, cyclohexyl, aryl, acetoxymethyl, pivaloyloxymethyl, methoxymethyl or 4-methylene-5-methyl-1,3-dioxolene-2-one group, a sodium, potassium, silver, ammonium or $C_{1-4}$ tertiary or quarternary alkyl ammonium cation; and $R_3$ is a hydroxy, hydroxymethyl, amino, aminomethyl, $C_{1-4}$ alkylamino, $C_{1-4}$ alkoxycarbonyl, formylamino or $RCH(NH_2)CONH-$ group wherein R is a hydrogen atom, an optionally substituted $C_1$-$C_4$ alkyl group or a phenyl group.

More preferred compounds included in the present invention are;

7-(1-amino-5-azaspiro[2,4]heptane-5-yl)-1-cyclopropyl-6,8-difluoro-1,4-dihydro-4-oxoquinoline-3-carboxylic acid;

7-(1-amino-5-azaspiro[2,4]heptane-5-yl)-1-cyclopropyl-6,8-difluoro-1,4-dihydro-4-oxoquinoline-3-carboxylic acid lactate;

7-(1-amino-5-azaspiro[2,4]heptane-5-yl)-1-cyclopropyl-6,8-difluoro-1,4-dihydro-4-oxoquinoline-3-carboxylic acid hydrochloride;

7-(1-amino-5-azaspiro[2,4]heptane-5-yl)-1-cyclopropyl-6-fluoro-1,4-dihydro-4-oxoquinoline-3-carboxylic acid;

7-(1-amino-5-azaspiro[2,4]heptane-5-yl)-1-cyclopropyl-6-fluoro-1,4-dihydro-4-oxoquinoline-3-carboxylic acid lactate;

7-(1-amino-5-azaspiro[2,4]heptane-5-yl)-1-cyclopropyl-6-fluoro-1,4-dihydro-4-oxoquinoline-3-carboxylic

hydrochloride;

7-(1-amino-5-azaspiro[2,4]heptane-5-yl)-8-chloro-1-cyclopropyl-6-fluoro-1,4-dihydro-4-oxoquinoline-3-carboxylic acid;

7-(1-amino-5-azaspiro-[2,4]heptane-5-yl)-8-chloro-1-cyclopropyl-6-fluoro-1,4-dihydro-4-oxoquinoline-3-carboxylic acid lactate;

7-(1-amino-5-azaspiro[2,4]heptane-5-yl)-1-cyclopropyl-6-fluoro-4-oxo-1,4,dihydro-1,8-naphthyridine-3-carboxylic acid;

7-(1-amino-5-azaspiro[2,4]heptane-5-yl)-5-amino-1-cyclopropyl-6,8-difluoro-oxoquinoline-3-carboxylic acid;

7-(1-amino-5-azaspiro[2,4]heptane-5-yl)-5-amino-1-cyclopropyl-6,8-difluoro-1,4-dihydro-4-oxoquinoline-3-carboxylic acid lactate;

1-cyclopropyl-6,8-difluoro-7-(1-methylamino-5-azaspiro [2,4]heptane-5-yl)-1,4-dihydro-4-oxoquinoline-3-carboxylic acid;

8-chloro-1-cyclopropyl-6-fluoro-7-(1-methylamino-5-azaspiro[2,4]heptane-5-yl)-1,4-dihydro-4-oxoquinoline-3-carboxylic acid;

9-fluoro-3-(S)-methyl-10-(1-methylamino-5-azaspiro[2,4] heptane-5-yl)-7-oxo-2,3-dihydro-7H-pyrido-[1,2,3-de]-1,4-benzoxazine-6-carboxylic acid;

10-(1-amino-5-azaspiro[2,4]heptane-5-yl)-9-fluoro-3(S)-methyl-7-oxo-2,3-dihydro-7H-pyrido[1,2,3-de]-1,4-benzoxazine-6-carboxylic acid;

1-cyclopropyl-6-fluoro-7-(1-methylamino-5-azaspiro[2,4] heptane-5-yl)-1,4-dihydro-4-oxoquinoline-3-carboxylic acid;

5-amino-1-cyclopropyl-6,8-difluoro-7-(1-methylamino-5-azaspiro[2,4]heptane-5-yl)-1,4-dihydro-4-oxoquinoline-3-carboxylic acid;

7-(1-aminomethyl-5-azaspiro[2,4]heptane-5-yl)-1-cyclopropyl-6,8-difluoro-1,4-dihydro-4-oxoquinoline-3-carboxylic acid;

7-(1-amino-5-azaspiro[2,5]octane-5-yl)-1-cyclopropyl-6,8-difluoro-1,4-dihydro-4-oxoquinoline-3-carboxylic acid;

7-(1-amino-5-azaspiro[2,5]octane-5-yl)-8-chloro-6-fluoro-1-cyclopropyl-1,4-dihydro-4-oxoquinoline-3-carboxylic acid;

1-cyclopropyl-6,8-difluoro-7-(1-hydroxymethyl-5-azaspiro [2,4]heptane-5-yl)-1,4-dihydro-4-oxoquinoline-3-carboxylic acid;

7-(1-amino-5-azaspiro[2,4]heptane-5-yl)-1-cyclopropyl-6-fluoro-8-methoxy-1,4-dihydro-4-oxoquinoline-3-carboxylic acid;

7-(1-amino-5-azaspiro[2,4]heptane-5-yl)-1-cyclopropyl-6-fluoro-5-methyl-1,4-dihydro-4-oxoquinoline-3-carboxylic acid;

7-(1-amino-5-azaspiro[2,4]heptane-5-yl)-1-cyclopropyl-6-fluoro-5-methyl-4-oxo-1,4-dihydro-1,8-naphthyridine-3-carboxylic acid;

7-(1-t-butoxycarbonylamino-5-azaspiro[2,4]heptane-5-yl)-1-cyclopropyl-6,8-difluoro-1,4-dihydro-4-oxoquinoline-3-carboxylic acid;

7-(1-t-butoxycarbonylamino-5-azaspiro[2,4]heptane-5-yl)-1-cyclopropyl-6,8-difluoro-1,4-dihydro-4-oxoquinoline-3-carboxylic acid ethylester;

7-(1-amino-5-azaspiro[2,4]heptane-5-yl)-1-cyclopropyl-6,8-difluoro-1,4-dihydro-4-oxoquinoline-3-carboxylic acid ethylester hydrochloride;

7-(1-acetamino-5-azaspiro[2,4]heptane-5-yl)-1-cyclopropyl-6,8-difluoro-1,4-dihydro-4-oxoquinoline-3-carboxylic acid;

7-(1-amino-acetylamino-5-azaspiro[2,4]heptane-5-yl)-1-cyclopropyl-6,8-difluoro-1,4-dihydro-4-oxoquinoline-3-carboxylic acid;

7-[1-(2-amino-3-methylbutanoyl)-amino-5-azaspiro[2,4] heptane-5-yl)-1-cyclopropyl-8-chloro-1,4-dihydro-4-oxoquinoline-3-carboxylic acid;

8-chloro-1-cyclopropyl-7-[1-(N-formyl-N-methyl)-amino-5-azasprio[2,4]heptane-5-yl)-6-fluoro-1,4-dihydro-4-oxoquinoline-3-carboxylic acid;

7-(1-amino-5-azaspiro[2,4]heptane-5-yl)-1-ethyl-6,8-difluoro-1,4-dihydro-4-oxoquinoline-3-carboxylic acid;

7-(1-amino-5-azaspiro[2,5]octane-5-yl)1-cyclopropyl-6-fluoro-4-oxo-1,4-dihydro-1,8-naphthyridine-3-carboxylic acid;

7-(1-amino-5-azaspiro[2,4]heptane-5-yl)-1-t-butyl-6-fluoro-1,4-dihydro-1,4-oxoquinoline-3-carboxylic acid;

7-(1-amino-5-azaspiro[2,4]heptane-5-yl)-1-(4-fluorophenyl)-6,8-difluoro-1,4-dihydro-1,4-oxoquinoline-3-carboxylic acid;

7-(1-amino-5-azaspiro[2,4]heptane-5-yl)-1-(2,4-diflorophenyl)-6-fluoro-1,4-dihydro-4-oxoquinoline-3-

carboxylic acid;

7-(1-amino-5-azaspiro[2,4]heptane-5-yl)-1-(2,4-difluorophenyl)-6-fluoro-5-methyl-1,4-dihydro-1,4-oxoqininoline-3-carboxylic acid;

7-(1-amino-5-azaspiro[2,4]heptane-5-yl)-1(2,4-difluorophenyl)-6-fluoro-5-methyl-4-oxo-1,4-dihydro-1,8-naphthyridine-3-carboxylic acid;

7-(1-amino-5-azaspiro[2,4]heptane-5-yl)-1(1,2-cis-2-fluoro-1-cyclopropyl)-6,8-difluoro-1,4-dihydro-4-oxoquinoline-3-carboxylic acid;

7-(1-amino-5-azaspiro[2,4]heptane-5-yl)-1-(1,2-cis-2-fluoro-1-cyclopropyl)-6-fluoro-1,4-dihydro-4-oxoquinoline-3-carboxylic acid;

7-(1-amino-5-azaspiro[2,4]heptane-5-yl)-1-(1,2-cis-2-fluoro-1-cyclopropyl)-8-chloro-6-fluoro-1,4-dihydro-4-oxoquinoline-3-carboxylic acid;

7-(1-(R)-amino-3(R)-5-azaspiro[2,4]heptane-5-yl)-1-cyclopropyl)-6,8-difluoro-1,4-dihydro-4-oxoquinoline-3-carboxylic acid;

7-(1-(R)-amino-3(S)-5-azaspiro[2,4]heptane-5-yl)-1-cyclopropyl)-6,8-difluoro-1,4-dihydro-4-oxoquinoline-3-carboxylic acid;

7-(1-(S)-amino-3(S)-5-azaspiro[2,4]heptane-5-yl)-1-cyclopropyl)-6,8-difluoro-1,4-dihydro-4-oxoquinoline-3-carboxylic acid; and

7-(1(S)-amino-3(S)-5-azaspiro[2,4]heptane-5-yl)-1-cyclopropyl-6,8-difluoro-1,4-dihydro-4-oxoquinoline-3-carboxylic acid.

The quinolone compounds of the present invention represented by formula(I) and pharmaceutically acceptable salts thereof can be prepared by employing the following processes.

Process A

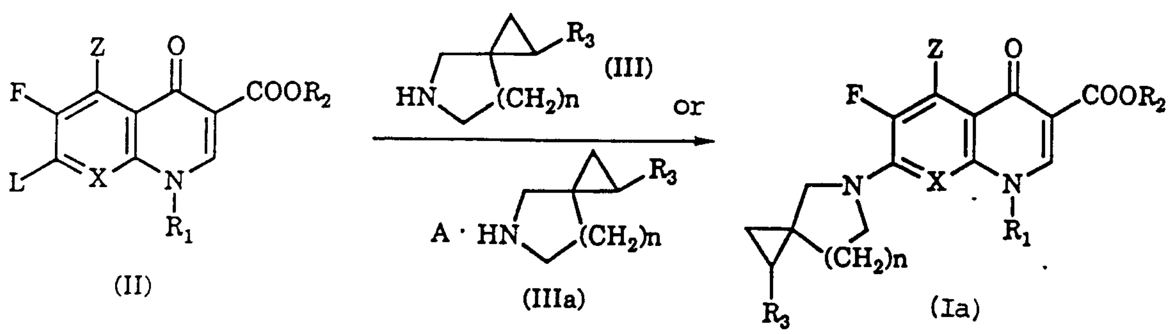

(II) (III) or (IIIa) (Ia)

wherein:

$R_1$, $R_3$, X, Z and n are the same as defined previously;

$R_2$ is a hydrogen atom or a carboxy protecting group;

L is a leaving group which is a halogen, e.g. fluorine, chlorine or bromine atom or an ethanesulfonyl, benzenesulfonyl or p-toluenesulfonyl group; and

A is a strong acid such as hydrochloric, hydrobromic or sulfuric acid.

In accordance with the above Process A, the quinolone compounds of formula(I) wherein $R_2$ is a hydrogen atom or a carboxy protecting group(hereinafter referred to as the compounds of formula(Ia)) can be prepared by condensing the compounds of formula (II) with the compounds of formula(III) or (IIIa) in the presence of a solvent.

The compounds of formula(II) employed as starting material are commercially available; and the compounds of formula(III) or (IIIa) are novel and can be prepared in accordance with the methods described in Korean Patent Application No. 91-25885 entitled "Novel azaspiro derivatives and processes for preparing same" (corresponding to WO-A ...........).

The stereochemical configuration of the compounds of formula(III) or (IIIa) may be (1,3)-(R,S), (1,3)-(R,R), (1,3)-(S,S) or (1,3)-(S,R).

The preferred solvent used in the above condensation reacton is an inert solvent such as benzene, toluene, tetrahydrofuran, dioxane, dimethylformamide, dimethylsulfoxide, sulforane, lower alcohol, acetonitrile or pyridine. A base such as sodium hydride, sodium hydroxide, potassium hydroxide, sodium carbonate, potassium carbonate, triethylamine, diisopropylethylamine or 1,8-diazabicyclo[5,4,0]undec-7-ene may also be used to neutralize the acids produced in the reaction or to convert the salts of formula (IIIa) into their free form.

The above condensation reaction may be carried out at a temperature between 10 and 150°C for 10 min to 24 hours; and the compounds of formula(II) and the compounds of formula(III) or (IIIa) are used at the molar ratio of 1:1 to 1:5, more preferably 1:1.1 to 1:1.5.

The compounds of formula(Ia) obtained from Process A wherein $R_2$ is a hydrogen atom(hereinafter referred to as the compounds of formula(Ia-1)) can be converted to the compounds of formula(Ia) wherein $R_2$ is a carboxyl protecting group(hereinafter referred to as the compounds of formula(Ia-2)) by the following Process B.

Process B

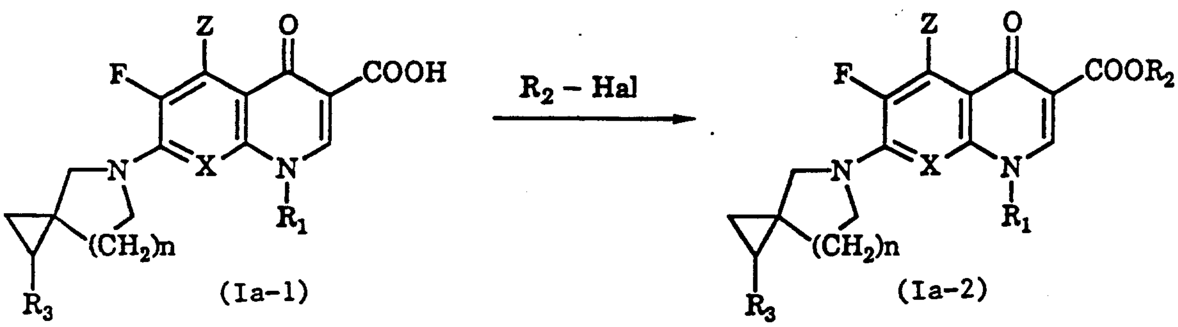


(Ia-1) (Ia-2)

wherein:

$R_1$, X, Z and n are the same as defined previously;
$R_2$ is a carboxyl protecting group;
$R_3$ is the same as defined previously, excepting an amino or monoalkyl amino group; and
Hal is a chlorine, iodine or bromine atom.

In accordance with the above Process B, the compounds of formula(Ia-2) can be prepared by reacting the compounds of formula (Ia-1) with the compounds of formula $R_2$-Hal in a solvent, preferably in the presence of a base at a temperature ranging between a room temperature and 120°C.

The preferred solvent used in the reaction is benzene, toluene, dichloromethane, dimethylformamide, dimethylsulfoxide, dioxane or tetrahydrofuran, etc; and the base which may be used is triethylamine, diisopropylethylamine, 1,8-diazabicyclo[5,4,0]undecene, sodium carbonate, potassium carbonate or potassium hydroxide.

The compounds of formula(Ia-2) obtained from Process A can be also converted to the compounds of formula(Ia-1) by an alkali or acid hydrolysis or a reduction with hydrogen in accordance with the following Process C.

Process C

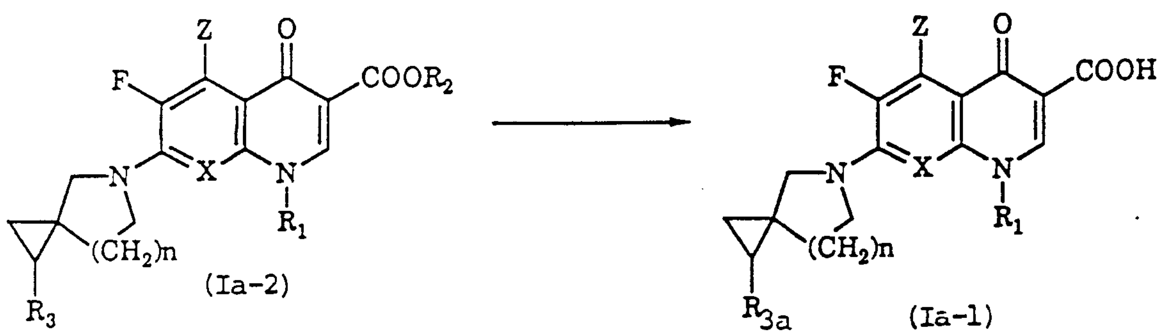

(Ia-2) (Ia-1)

wherein:

$R_1$, $R_3$, X, Z and n are the same as defined previously;
$R_2$ i s a carboxy protecting group; and
$R_{3a}$ is a group of $-(CH_2)_m-Y$ wherein m is 0 or 1 and Y is a hydroxy group or an amino group

optionally substituted with one or two $C_1$-$C_4$ alkyl radicals.

In accordance with the above Process C, the compounds of formula(Ia-1) can be prepared by treating the compounds of formula (Ia-2) with sodium hydroxide, potassium hydroxide, sodium carbonate, potassium carbonate, hydrochloric, hydrobromic or sulfuric acid, or Palladium-charcoal or Palladium hydroxide under hydrogen. The prefered solvent used in this reaction is tetrahydrofuran, methanol or ethanol, water or a mixture thereof. This reaction is preferably carried out at a temperature ranging between room temperature and 100 °C.

The compounds of formula(Ia) obtained by Process A wherein $R_3$ is $(CH_2)_m$-$NHR_4$(hereinafter referred to as the compounds of formula(Ia-3)) can be converted to the compounds of formula(I) wherein $R_3$ is $(CH_2)_m$-$NR_4R_5$(hereinafter referred to as the compounds of formula(Ia-4)) by the following Process D.

Process D

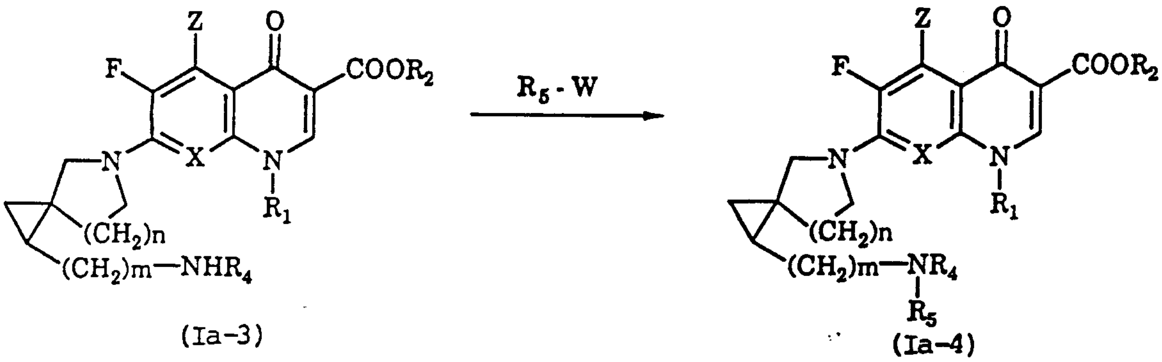


(Ia-3) (Ia-4)

wherein:

$R_1$, X, Z and n are the same as defined previously;

$R_2$ is a hydrogen atom or a carboxy protecting group;

m is 0 or 1;

$R_4$ is a hydrogen atom or a $C_1$-$C_4$ alkyl group; and

$R_5$ is a nitrogen-protecting group such as CN, 4-methylene-5-methyl-1,3-dioxolene-2-one or $(CH_2)_pCO$-$R_6$ wherein p is 0, 1 or 2 and $R_6$ is a hydrogen atom, an amino group, an aminoalkyl group wherein alkyl group is methyl, ethyl, propyl, isopropyl, butyl, isobutyl, pentyl, isopentyl or phenylethyl which is derived from amino acid, and amino group can be optionally protected with t-butoxycarbonyl, a straight or branched $C_{1-6}$ alkyl or alkoxy group optionally substituted with methoxy, ethoxy, fluoro or hydroxy radical, a $C_6$ or $C_{10}$ aryl group optionally substituted with methyl, methoxy or nitro radical or a straight or branched alkyl group optionally substituted with 1 to 5 substituents selected from a fluorine or chlorine atom, a hydroxy, nitro,amino and carboxy radical or a phenyl group.

In accordance with the above Process D, the compounds of formula(Ia-4) can be prepared by reacting the compounds of formula (Ia-3) with the compounds of $R_5$-W in a solvent such as dimethylsulfoxide, dimethylformamide, tetrahydrofuran, sulfolane, dioxane, pyridine or haloalkane or a mixture thereof at a temperature ranging from 0 to 140 °C, preferably 10 to 100 °C. A conventional inorganic or organic base, preferably alkaline metal hydroxide, alkaline metal carbonate or tertiary amine, e.g., pyridine, triethylamine, diisopropylethylamine or 1,8-diazabicyclo[5,4,0]undec-7-ene(DBU) may be used to accelerate the above reaction.

The compounds of formula(Ia-3) nay be reacted with the compounds of $R_5$-W at the molar ratio of 1:1 to 1:4, preferably 1:1.1 to 1:1.5.

The compounds of $R_5$-W are well known in the art; and the examples are acetic formic anhydride, acetic anhydride, propionic anhydride, acetyl chloride, pentanoyl chloride, benzoylchloride, 3-chlorobenzoylchloride, 4-fluorobenzoylchloride, 4-nitrobenzoylchloride, 2-chloro-4-nitrobenzoylchloride, 4-methylbenzoylchloride, monomethylchloroformyl succinate, 4-nitrophenyl-N-(t-butoxycarbonyl)-glycine, 4-nitrophenyl-N-(t-butoxycarbonyl)-L-alanine, 2,3,5-trichlorophenyl-N-(t-butoxycarbonyl)-D-alanine 4-nitrophenyl-N-(t-butoxycarbonyl)-L-leucine, 4-nitrophenyl-N-(t-butoxycarbonyl)-L-valine, 3-methoxypropionyl chloride, methylchlorocarbonate, ethylchloro carbonate, n-butylchloro carbonate, diethyl carbonate, cyanogen chloride, diphenyl carbonate, cyanogen bromide, dimethyl dicarbonate, diethyl dicarbonate, di-t-butyldicarbonate, etc.

The compounds of formula(Ia) can be converted to acid addition salts thereof with an organic or inorganic acid such as hydrochloric, hydrobromic, sulfuric, phosphoric, formic, acetic, trifluoroacetic, propionic, lactic, citric, succinic, maleic, malonic, fumaric, tartaric, ascorbic, glutaminic, methanesulfonic, benzenesulfonic or p-toluenesulfonic acid.

The compounds of formula(Ia) can also be converted to their base addition salts with an alkaline metal or alkaline earth metal such as sodium, potassium, calcium or magnesium; and a tertiary or quarternary $C_{1-4}$ alkylammonium.

The above conversion reaction comprises: dissolving or suspending the compounds of formula(Ia) in a solvent such as dichloromethane, chloroform or a mixture of dichloromethane with methanol, ethanol or water; adding thereto an aqueous or alcoholic solution of a selected acid or base; stirring the reaction solution at a temperature ranging between a room temperature and the boiling point of the solvent used; and, thereafter, collecting the produced precipitates or removing the solvent under a reduced pressure.

The compounds of formula(I) further encompass the hydrates of the acid or base addition salts.

The quinolone compounds of the present invention are effective against bacteria and bacteroids; and thus believed to be useful for protection or treatment by chemotherapy against a local or systemic infection in the human or animal bodies of said microorganisms.

The compounds of the present invention can be administered topically, orally, parenterally or rectally, preferably intravenously or intramuscularly. In general, it has been shown advantageous to administer the compounds of the present invention in an amount of about 0.5 to 500, preferably 5 to 100mg/kg of body weight per day in single or divided doses. A daily dosage ranging from about 1 to 200mg/kg of body weight, particularly 1 to 50mg/kg of body weight is preferred. However, it will be understood that the amount of the compound actually administered may be adjusted in the light of the relevant circumstances including the form of formulation, the chosen route of administration, the age, weight and response of the individual patient, and the severity of the patient's symptoms.

One or more compounds of the present invention may be either administered as such or formulated for administration by mixing therewith non-toxic, inert pharmaceuticallY acceptable excipients such as solid, semi-solid or liquid diluent, filler and carrier. Examples of such formulation are in the form of: a tablet, lozenge, capsule, granule, suppository, solution, suspension, emulsion, paste, ointment, cream, lotion, powder, spray and the like.

In case of tablet, lozenge, capsule and granule, the active compounds of the present invention may be combined with conventional excipients, e.g., fillers and extenders such as starch, lactose, sucrose, glucose mannitol and the like, binders such as carboxymethyl cellulose, alginate, gelatine, polyvinylpyrolidone and the like; disintegrants such as calcium carbonate, sodium bicarbonate and the like; absorption accelerants such as quarternary ammonium compound and the like; wetting agents such as cetyl alcohol, glycerine monostearate and the like; adsorbents such as kaoline, bentonite and the like; lubricants such as talc, calcium stearate, magnesium stearate, solid polyethylene glycol and the like; or mixtures thereof. The tablet, lozenge, capsule, pill and granule may be coated with conventional coating materials including any opacifier.

The suppository may contain conventional soluble or insoluble excipients, e.g., polyethylene glycol, fat, polymeric ester or a mixture thereof in addition to the active compound.

The ointment, paste, cream and the like may contain conventional excipients, e.g., animal or vegetable fat, wax, paraffin, starch, cellulose derivatives, polyethylene glycol, bentonite, talc, zine oxide or a mixture thereof in addition to the active compounds.

The solution or emulsion for oral administration may contain conventional excipients such as solvents, solubilizers and emulsifiers, e.g., water, ethyl alcohol, benzyl benzoate, propylene glycol, cotton seed oil, pinutts oil, corn oil, olive oil, fatty esters such as glycerine, polyethylene glycol or sorbitan, or a mixture thereof in addition to the active compounds.

The solution, suspension or emulsion for parenteral administration may contain a sterilized isometric solution or emulsion. In particular, the suspension may contain the excipients e.g., liquid diluent or suspending agent such as water, ethyl alcohol or propylene glycol.

The formulations may comprise about 0.1 to 99.5% by weight, preferably 0.5 to 95% by weight of the active compounds of the present invention; and may further contain conventionally accepted amounts of dyes, preservatives, sweeteners and additives.

It will be apparent to those skilled in the art that certain changes and modifications may be made to this invention without departing from the spirit or scope of the invention as it is further illustrated below.

Example 1: Preparation of 7-(1-amino-5-azaspiro[2,4]heptane-5-yl)-1-cyclopropyl-6,8-difluoro-1,4-dihydro-4-oxoquinoline-3-carboxylic acid

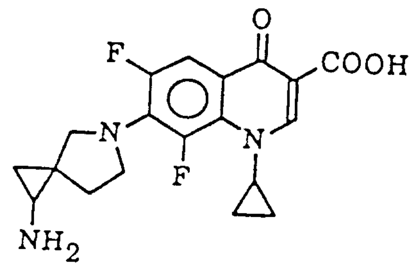

1.28g(4.52mmol) of 1-cyclopropyl-6,7,8-trifluoro-1,4-dihydro-4-oxoquinoline-3-carboxylic acid, 1g-(5.40mmol) of 1-amino-5-azaspiro[2,4]heptane hydrochloride and 2.4g(15.76mmol) of 1,8-diazabicyclo-[5,4,0]undec-7-ene were dissolved in 10mℓ of acetonitrile; and the resulting solution was refluxed for 3 hours and stirred for 1 hour. The precipitate produced was filtered, washed with chilled acetonitrile, water and ethylether in turn and dried under a reduced pressure to obtain 1g of the desired compound (yield: 58.9%).

m.p.: 217°C

$^1$H-NMR (DMSO-$d_6$, δ ppm) : 8.59 (1H, s), 7.68 (1H, dd, J = 14.1, 1.6 Hz), 2.28 (1H, dd, J = 7.2, 4.8 Hz), 1.86 (1H, m), 1.67 (1H, m), 1.15 (4H, m), 0.78 (1H, dd, J=7.2, 4.5 Hz), 0.37 (1H, t, J=4.5 Hz)

Example 2: Preparation of 7-(1-amino-5-azaspiro[2,4]heptane-5-yl)-1-cyclopropyl-6,8-difluoro-1,4-dihydro-4-oxoquinoline-3-carboxylic acid lactate

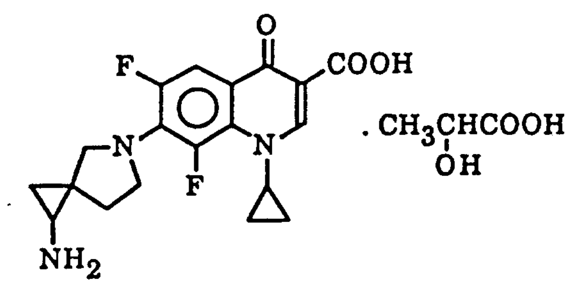

0.45g(1.2mmol) of 7-(1-amino-5-azaspiro[2,4]heptane-5-yl)-1-cyclopropyl-6,8-difluoro-1,4-dihydro-4-oxoquinoline-3-carboxylic acid prepared in Example 1 was dissolved in a solvent containing 5mℓ of chloroform and 5mℓ of methanol; and the resulting solution was well mixed with 0.12g of 85% lactic acid. The reaction mixture was then concentrated under a reduced pressure at a temperature lower than 40°C; and 50mℓ of ethylether was added to obtain the powdery material. The resultant was filtered and dried under a reduced pressure to obtain 5.2g of the desired compound(yield: 93.1%).

m.p: 150~152°C

$^1$H-NMR (DMSO-$d_6$, δ ppm) : 8.81 (1H, s), 7.82 (1H, d, J = 13.7 Hz), 4.35 (1H, m), 3.99 (1H, q, J = 7 Hz), 3.73 (4H, m), 3.67 (4H, m), 2.36 (1H, dd, J = 7.2, 4.8 Hz), 1.83 (1H, m), 1.76 (1H, m), 1.15 (3H, d, J = 7 Hz), 0.91 (4H, m), 0.80 (1H, dd, J = 7.2, 4.5 Hz), 0.45 (1H, t, J = 4.5 Hz).

Example 3: Preparation of 7-(1-amino-5-azaspiro[2,4]heptane-5-yl)-8-chloro-1-cyclopropyl-6-fluoro-1,4-dihydro-4-oxoquinoline-3-carboxylic acid

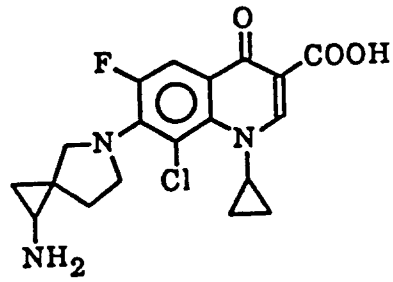

1.8g(6.0mmol) of 8-chloro-1-cyclopropyl-6,7-difluoro-1,4-dihydro-4-oxoquinoline-3-carboxylic acid, 1.3g-(7.0mmol) of 1-amino-5-azaspiro[2,4]heptane hydrochloride and 3.2g(21.0mmol) of 1,8-diazabicyclo[5,4,0]-undec-7-ene were dissolved in 10mℓ of acetonitrile; and the resulting solution was refluxed for 3 hours and concentrated under a reduced pressure. 20mℓ of chloroform was added thereto; and the resultant solution was washed with water; The organic layer was dried over magnesium sulfate, concentrated under a reduced pressure, crystallized with ethanol-ethylacetate mixture and filtered. The filtrate was washed with acetonitrile, water and ethylether in turn and dried under a reduced pressure to obtain 1.0g of the desired compound-(yield: 42.4%).

m.p.: 158~160 ° C

$^1$H-NMR (DMSO-$d_6$, $\delta$ ppm) : 8.79 (1H, s), 7.81 (1H, d, J = 13.7 Hz), 6.35 (1H, br), 4.38 (1H, m), 3.42 (4H, m), 2.31 (1H, dd, J = 7.2, 4.8 Hz), 1.85 (1H, m), 1.75 (1H, m), 1.16 (2H, m), 0.93 (2H, m), 0.78 (1H, dd, J = 7.2, 4.5 Hz), 0.49 (1H, t, J = 4.5 Hz).

Example 4: Preparation of 7-(1-amino-5-azaspiro[2,4]heptane-5-yl)-8-chloro-1-cyclopropyl-6-fluoro-1,4-dihydro-4-oxoquinoline-3-carboxylic acid lactate

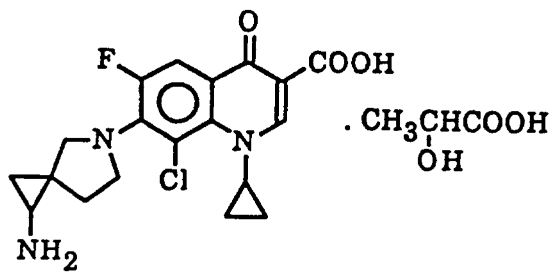

0.45g(1.15mmol) of 7-(1-amino-5-azaspiro[2,4]heptane-5-yl)-8-chloro-1-cyclopropyl-6-fluoro-1,4-dihydro-4-oxoquinoline-3-carboxylic acid prepared in Example 3 was dissolved in a solvent containing 15mℓ of chloroform and 5mℓ of methanol; and the resulting solution was well mixed with 0.11g of 85% lactic acid. The reaction mixture was then concentrated under a reduced pressure at a temperature lower than 40 ° C; and 5mℓ of ethylether was added to obtain the powdery material. The resultant was filtered and dried under a reduced pressure to obtain 0.53g of the desired compound (yield: 95.8%).

m.p.: 138~140 ° C

$^1$H-NMR(DMSO-$d_6$, $\delta$ ppm) : 8.59 (1H, s), 7.68 (1H, d, J = 13.7 Hz), 4.07 (1H, m), 3.99 (1H, q, J = 7 Hz), 3.82 (3H, m), 3.67 (1H, m), 2.28 (1H, dd, J = 7.2, 4.8 Hz), 1.83 (1H, m), 1.76 (1H, m), 1.15 (3H, d, J = 7 Hz), 0.91 (4H, m), 0.80 (1H, dd, J = 7.2, 4.5 Hz), 0.45 (1H, t, J = 4.5 Hz).

Example 5: Preparation of 7-(1-amino-5-azaspiro[2,4]heptane-5-yl)-1-cyclopropyl-6-fluoro-1,4-dihydro-4-oxoquinoline-3-carboxylic acid

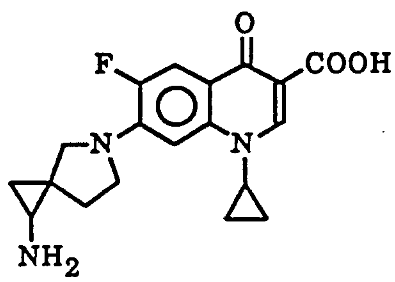

0.31g(1.17mmol) of 1-cyclopropyl-6,7-difluoro-1,4-dihydro-4-oxoquinoline-3-carboxylic acid, 0.26g-(1.40mmol) of 1-amino-5-azaspiro[2,4]heptane hydrochloride and 0. 53g(3.48mmol) of 1,8-diazabicyclo-[5,4,0]undec-7-ene were dissolved in 5mℓ of acetonitrile; and the resulting solution was refluxed for 3 hours and cooled to room temperature. The solid produced was filtered, washed with chilled acetonitrile, water and ethylether in turn and dried under a reduced pressure to obtain 0.30g of the desired compound(yield: 71.8%).

m.p.: 222~224° C

$^1$H-NMR (DMSO-$d_6$, δ ppm): 8.55 (1H, s), 7.56 (1H, d, J = 14.2 Hz), 7.02 (1H, d, J = 7.76 Hz), 3.81 (1H, m), 3.68 (3H, m), 3.53 (1H, m), 2.28 (1H, dd, J = 72, 4.8 Hz), 1.98 (1H, m), 1.70 (1H, m), 1.27 (2H, m), 1.11 (2H, m), 0.79 (1H, dd, J = 7.2, 4.5 Hz), 0.38 (1H, t, J = 4.5 Hz).

Example 6: Preparation of 7-(1-amino-5-azaspiro[2,4]heptane-5-yl)-1-cyclopropyl-6-fluoro-4-oxo-1,4-dihydro-1,8-naphthyridine-3-carboxylic acid

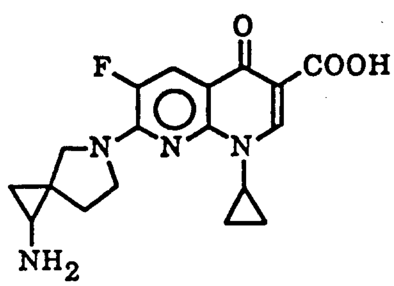

0.26g(0.98mmol) of 1-cyclopropyl-7-chloro-6-fluoro-4-oxo-1,4-dihydro-1,8-naphthyridine-3-carboxylic acid, 0.19g(1.03mmol) of 1-amino-5-azaspiro[2,4]heptane hydrochloride and 0.43g(2.82mmol) of 1,8-diazabicyclo[5,4,0]undece-7-ene were dissolved in 5mℓ of acetonitrile; and the resulting solution was refluxed for 3 hours and cooled to room temperature. The solid produced was filtered, washed with chilled acetonitrile, water and ethylether in turn and dried under a reduced pressure to obtain 0.15g of the desired compound (yield: 44.7%).

m.p.: 205~207° C

$^1$H-NMR (DMSO-$d_6$, δ ppm) : 8.52 (1H, s), 7.91 (1H, d, J = 12.8 Hz), 3.95 (1H, m), 3.72 (4H, m), 2.29 (1H, dd, J = 7.2, 4.8 Hz), 1.98 (1H, m), 1.71 (1H, m), 1.19 (2H, m), 1.08 (2H, m), 0.81 (1H, dd, J = 7.2, 4.5 Hz).

Example 7: Preparation of 7-(1-amino-5-azaspiro[2,4]heptane-5-yl)-1-cyclopropyl-5-amino-6,8-difluoro-1,4-dihydro-4-oxo-quinoline-3-carboxylic acid

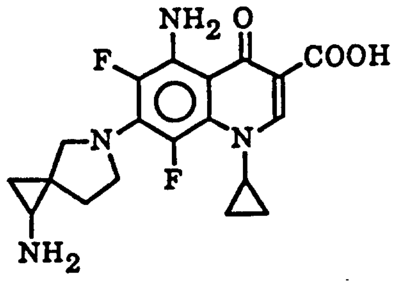

0.1g(0.33mmol) of 5-amino-1-cyclopropyl-6,7,8-trifluoro-1,4-dihydro-4-oxoquinoline-3-carboxylic acid, 74mg(0.40mmol) of 1-amino-5-azaspiro[2,4]heptane hydrochloride and 0.18g(1.2mmol) of 1,8-diazabicyclo-[5,4,0]undec-7-ene were dissolved in 2mℓ of acetonitrile; and the resulting solution was refluxed for 4 hours and cooled to room temperature. The solid produced was filtered, washed with chilled acetonitrile, water and ethylether in turn and dried under a reduced pressure to obtain 62mg of the desired compound in yellow color(yield: 48%).

$^1$H-NMR (DMSO-$d_6$, $\delta$ ppm) : 8.41 (1H, s), 7.10 (2H, br. s), 3.98 (1H, m), 3.65~3.43 (4H, m), 2.55 (1H, dd, J = 7.2, 4.8 Hz), 1.86 (1H, m), 1.69 (1H, m), 1.10 (4H, m), 0.83 (1H, dd, J = 7.2, 4.5 Hz), 0.49 (1H, t, J = 4.5 Hz).

Example 8: Preparation of 7-(1-methylamino-5-azaspiro[2,4]heptane-5-yl)-1-cyclopropyl-6,8-difluoro-1,4-dihydro-4-oxo-quinoline-3-carboxylic acid

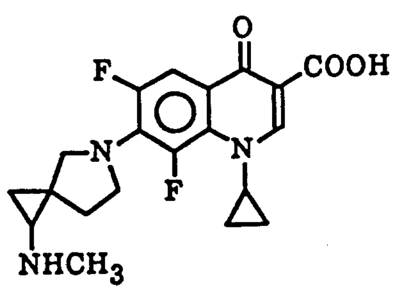

0.6g(2.1mmol) of 1-cyclopropyl-6,7,8-trifluoro-1,4-dihydro-4-oxoquinoline-3-carboxylic acid, 0.5g-(2.5mmol) of 1-methylamino-5-azaspiro[2,4]heptane hydrochloride and 0.96g(6.3mmol) of 1,8-diazabicyclo-[5,4,0]undec-7-ene were dissolved in 3mℓ of acetonitrile; and the resulting solution was refluxed for 3 hours and cooled to room temperature. The solid produced was filtered, washed with chilled acetonitrile, water and ethylether in turn and dried under a reduced pressure to obtain 0. 45g of the desired compound(yield: 54%).

m.p.: 204~207 °C

$^1$H-NMR ($CDCl_3$ + $CD_3COOD$, $\delta$ ppm) : 7.3 (1H, s), 7.75 (1H, d, J = 14.2 Hz), 4.29 (1H, m), 4.29 (1H, m), 3.89 (2H, m), 3.56 (2H, m), 2.0 (3H, s), 2.38 (1H, m), 1.27~0.95 (4H, m), 0.85 (1H, dd, J = 7.2, 4.5 Hz), 0.57 (1H, t, J = 4.5 Hz)

Example 9: Preparation of 7-(1-methylamino-5-azaspiro[2,4]heptane-5-yl)-8-chloro-1-cyclopropyl-6-fluoro-1,4-dihydro-4-oxo-quinoline-3-carboxylic acid

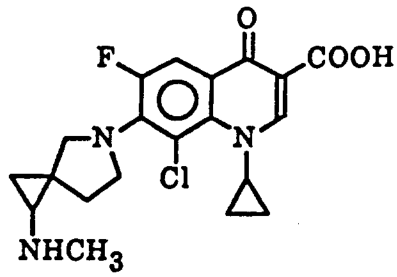

0.23g(0.77mmol) of 8-chloro-1-cyclopropyl-6,7-difluoro-1,4-dihydro-4-oxoquinoline-3-carboxylic acid, 0.18g(0.93mmol) of 1-methylamino-5-azaspiro[2,4]heptane hydrochloride and 0.41g(2.69mmol) of 1,8-diazabicyclo[5,4,0]undec-7-ene were dissolved in 3mℓ of acetonitrile; and the resulting solution was refluxed for 4 hours. The reaction mixture was then cooled to room temperature and concentrated under a reduced pressure. The resulting material was combined with 10mℓ of chloroform, washed with water several times and dried over magnesium sulfate. The residue was fractionated on silica gel column chromatography-(chloroform: methanol: water = 15:3:1) to obtain 0.15g of the desired compound(yield: 48%).

m.p.: 225~227 °C

$^1$H-NMR ($CDCl_3$, δ ppm) : 8.83 (1H, s), 7.93 (1H, d, J = 13.2 Hz), 4.28 (1H, m), 3.91 (2H, m), 3.57 (2H, m), 2.41 (3H, s), 2.12 (1H, m), 1.28-0.94 (4H, m), 0.87 (1H, dd, J = 7.2, 4.5 Hz), 0.58 (1H, t, J = 4.5 Hz)

Example 10: Preparation of 9-fluoro-3-(S)-methyl-10-(1-methylamino-5-azaspiro[2,4]heptane-5-yl)-7-oxo-2,3-dihydro-7H-pyrido-[1,2,3-de]-1,4-benzoxazine-6-carboxylic acid

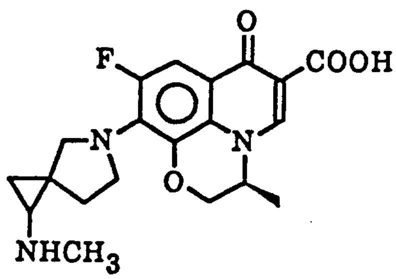

0.51g(1.8mmol) of 9,10-difluoro-3-(S)-methyl-7-oxo-2,3-dihydro-7H-pyrido-[1,2,3-de]-1,4-benzoxaine-6-carboxylic acid, 0.43g (2.2mmol) of 1-methylamino-5-azaspiro[2.4]heptane hydrochloride and 0.93g-(6.1mmol) of 1,8-diazabicyclo[5,4,0]undec-7-ene were dissolved in 5mℓ of acetonitrile; and the resulting solution was refluxed for 4 hours and concentrated under a reduced pressure. The resulting material was dissolved in 10mℓ of chloroform, washed with water several times, dried over magnesium sulfate and concentrated. The residue was fractionated on silica gel column chromatography (chloroform: methanol: water = 15:3:1) to obtain 0. 27g of the desired compound(yield: 39%).

m.p.: 240~243 °C

$^1$H-NMR ($CDCl_3$, δ ppm) : 8.56 (1H, s), 7.65 (1H, d, J = 14 Hz), 4.50~4.25 (3H, m), 3.97~3.68 (4H, m), 2.72 (1H, dd, J = 7.2, 4.8 Hz), 2.42 (3H, s), 2.01 (2H, m), 1.57 (3H, d, J = 6.7 Hz), 0.85 (1H, dd, J = 7.2, 4.5 Hz), 0.56 (1H, t, J = 4.5 Hz)

Example 11: Preparation of 7-(1-methylamino-5-azaspiro[2,4]heptane-5-yl)-1-cyclopropyl-6-fluoro-1,4-dihydro-4-oxoquinoline-3-carboxylic acid

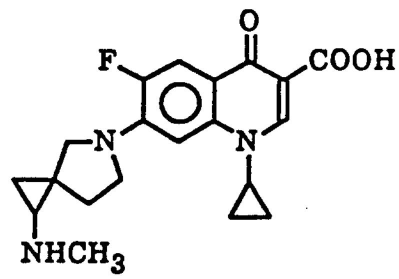

0.19g(0.72mmol) of 1-cyclopropyl-6,7-difluoro-1,4-dihydro-4-oxoquinoline-3-carboxylic acid, 0.17g-(0.86mmol) of 1-methylamino-5-azaspiro[2,4]heptane hydrochloride and 0.37g(2.43mmol) of 1,8-diazabicyclo[5,4,0]undec-7-ene were dissolved in 3mℓ of acetonitrile; and the resulting solution was refluxed for 3 hours and cooled to room temperature. The solid produced was filtered, washed with chilled acetonitrile, water and ethylether in turn and dried under a reduced pressure to obtain 0.15g of the desired compound(yield: 56%).

m.p.: 230~233 °C

$^1$H-NMR ($CDCl_3$, δ ppm) : 8.69 (1H, s), 7.92 (1H, d, J = 14.2 Hz), 6.81 (1H, d, J = 7.4 Hz), 4.29 (1H, m), 3.89 (2H, m), 3.56 (2H, m), 2.41 (3H, s), 2.12 (1H, m), 1.28~0.94 (4H, m), 0.85 (1H, dd, J = 7.2, 4.5 Hz), 0.57 (1H, t, J = 4.5 Hz)

Example 12: Preparation of 7-(1-methylamino-5-azaspiro[2,4]heptane-5-yl)-1-cyclopropyl-5-amino-6,8-difluoro-1,4-dihydro-4-oxoquinoline-3-carboxylic acid

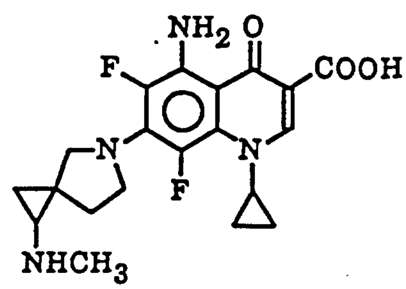

0.4g(1.34mmol) of 5-amnino-1-cyclopropyl-6,7,8-trifluoro-1,4-dihydro-4-oxoquinoline-3-carboxylic acid, 0.32g(1.6mmol) of 1-methylamino-5-azaspiro[2,4]heptane hydrochloride and 0.73g(4.8mmol) of 1,8-diazabicyclo[5,4,0]undec-7-ene were dissolved in 2mℓ of acetonitrile; and the resulting solution was refluxed for 2 hours and stirred at room temperature over night. The solid produced was filtered, washed with chilled acetonitrile, water and ethylether in turn and dried under a reduced pressure to obtain 0. 26g of the desired compound(yield: 48%).

m.p.: 212~215 °C

$^1$H-NMR (DMSO-$d_6$, δ ppm) : 8.65 (1H, s), 3.90 (1H, m), 3.85 (2H, m), 3.57 (2H, m), 2.41 (3H, s), 2.12 (1H, m), 1.28~0.93 (4H, m), 0.88 (1H, dd, J = 7.2, 4.5 Hz), 0.58 (1H, t, J = 4.5 Hz)

Example 13: Preparation of 10-(1-amino-5-azaspiro[2,4]heptane-5-yl)-3-(S)-methyl-7-oxo-2,3-dihydro-7H-pyrido-[1,2,3-de]-1,4-benzoxazine-6-carboxylic acid

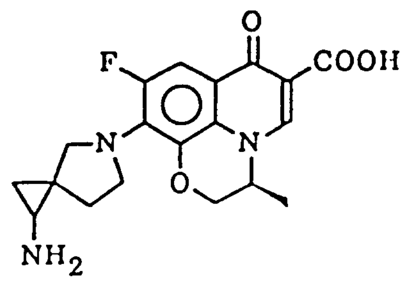

760mg of 9,10-difluoro-3-(S)-methyl-7-oxo-2,3-dihydro-7H-pyrido-[1,2,3-de]-1,4-benzoxazine-6-carboxylic acid and 543mg of 1-amino-5-azaspiro[2,4]heptane hydrochloride and 1.35mℓ of 1,8-diazabicyclo[5,4,0]-undec-7-ene were dissolved in 10mℓ of acetonitrile; and the resulting solution was refluxed for 5 hours, cooled and filtered. The residue was washed with acetonitrile to obtain 500mg of the desired compound-(yield: 50%).

m.p.: 195~198 °C

$^1$H-NMR (DMSO-$d_6$, δ ppm) : 8.89 (1H, s), 7.54 (1H, d, J = 14 Hz), 4.86 (1H, br, m), 4.53 (1H, br, m), 4.27 (1H, br, m), 3.95~3.4 (4H, m), 2.50 (1H, m), 1.95~1.75 (2H, m), 1.43 (3H, d, J = 6 Hz), 0.91 (1H, m), 0.69 (1H, m)

Example 14: Preparation of 10-(1-carboxylic acid-5-azaspiro[2,4] heptane-5-yl)-3-(S)-methyl-7-oxo-2,3-dihydro-7H-pyrido-[1,2,3-de]-1,4-benzoxazine-6-carboxylic acid

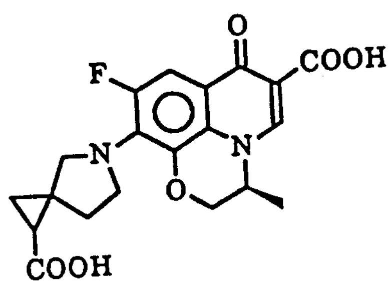

420mg of 9,10-difluoro-3-(S)-methyl-7-oxo-2,3-dihydro-7H-pyrido-[1,2,3-de]-1,4-benzoxazine-6-carboxylic acid and 250mg of 1-carboxylic acid-5-azaspiro[2,4]heptane hydrochloride and 691μℓ of 1,8-diazabicyclo-[5,4,0]undec-7-ene were dissolved in 3mℓ of acetonitrile; and the resulting solution was refluxed for 5 hours, cooled and filtered. The residue was washed with acetonitrile to obtain 300mg of the desired compound-(yield: 50%).

m.p.: 175~180 °C

Mass m/z (rel. int., %) : 402 (3), 374 (23), 330 (43), 247 (72), 237 (98), 222 (65), 140 (100), 126 (37), 98 (37)

$^1$H-NMR ($CDCl_3$ + $CD_3COOD$, δ ppm) : 9.64 (1H, s), 7.71 (1H, d, J = 14 Hz), 4.65~4.31 (3H, m), 4.20~3.45 (4H, m), 2.55 (1H, m), 1.85~1.7 (2H, m), 1.44 (3H, d, J = 6 Hz), 1.40~1.25 (2H, m)

Example 15: Preparation of 7-(1-aminoaethyl-5-azaspiro[2,4]heptane-5-yl)-1-cyclopropyl-6,8-difluoro-1,4-dihydro-4-oxoquinoline-3-carboxylic acid

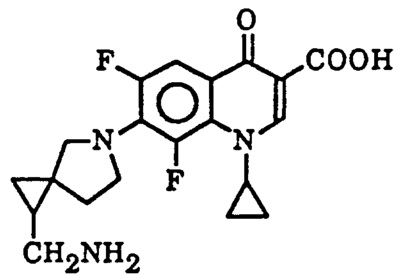

610mg of 1-aminomethyl-5-azaspiro[2,4]heptane diformate, 580mg of 1-cyclopropyl-6,7,8-trifluoro-1,4-dihydro-4-oxoquinoline-3-carboxylic acid and 750mg of 1,8-diazabicyclo[5,4,0]undec-7-ene were dissolved in 11mℓ of acetonitrile; and the resulting solution was heated under reflux and left at room temperature over night. The precipitate produced was filtered and washed with acetonitrile, water and ethylether in turn to obtain 570mg of the desired compound (yield: 69%).

Mass m/z (rel. int, %) : 389 ($M^+$, 32), 372 (62), 345 (85), 328 (100), 315 (23), 301 (40), 288 (75)

$^1$H-NMR ($CDCl_3$ + $CD_3COOD$, δ ppm) : 8.75, 8.69 (1H, two s), 7.75 (1H, d, J = 13.5 Hz), 4.05 (1H, m), 3.95~3.65 (4H, m), 3.20 (1H, m), 2.92 (1H, m), 2.05~1.85 (2H, m), 1.30~1.15 (4H, m), 1.00 (1H, dd, J = 8.5, 5.4 Hz), 0.70 (1H, t, J = 5.4 Hz)

Example 16: Preparation of 7-(1-amino-5-azaspiro[2,5]octane-5-yl)-1-cyclopropyl-6,8-difluoro-1,4-dihydro-4-oxoquinoline-3-carboxylic acid

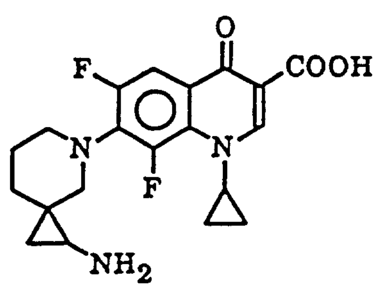

0.23g(1.2mmol) of 1-amino-5-azaspiro[2,5]octane hydrochloride, 0.28g(1mmol) of 1-cyclopropyl-6,7,8-trifluoro-1,4-dihydro-4-oxoquinoline-3-carboxylic acid and 0.46g(3mmol) of 1,8-diazabicyclo[5,4,0]undec-7-ene were dissolved in 3mℓ of acetonitrile; and the resulting solution was refluxed for 3 hours and cooled to room temperature. The precipitate produced was filtered, washed with acetonitrile, water and ethylether in turn and dried under a reduced pressure to obtain 0. 18g of the desired compound(yield: 40%).

$^1$H-NMR ($CDCl_3$, δ ppm) : 8.70 (1H, s), 7.71 (1H, d, J = 11.8 Hz), 3.98 (1H, m), 3.11 (1H, d, J = 12.5 Hz), 2.86 (1H, d, J = 12.5 Hz), 2.34 (1H, dd, J = 4.0, 7.3 Hz), 1.86~1.65 (4H, m), 1.32~1.12 (4H, m), 0.62 (1H, dd, J = 5.2, 7.3 Hz), 0.25 (1H, t, J = 4.5 Hz)

Example 17: Preparation of 7-(1-hydroxymethyl-5-azaspiro[2,4] heptane-5-yl)-1-cyclopropyl-6,8-difluoro-1,4-dihydro-4-oxoquinoline-3-carboxylic acid

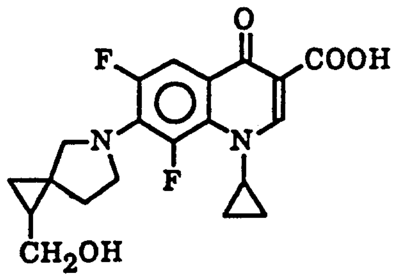

150mg of 1-hydroxymethyl-5-azaspiro[2,4]heptane, 283mg of 1-cyclopropyl-6,7,8-trifluoro-1,4-dihydro-4-oxoquinoline-3-carboxylic acid and 200μℓ of diisopropylethylamine were suspended in 10mℓ of acetonitrile; and the resulting solution was refluxed for 5 hours and left at a room temperature over night. The precipitate produced was filtered, washed with acetonitrile, water and ethylether in turn and dried under a reduced pressure to obtain 293mg of the desired compound(yield: 75%).

m.p.: 195~198 °C

$^1$H-NMR ($CDCl_3$ + $CD_3COOD$, δ ppm) : 8.70 (1H, s), 7.76 (1H, d, J = 13.5 Hz), 4.04 (1H, m), 3.90~3.62 (4H, m), 3.25 (1H, m), 2.80 (1H, m), 2.70~2.41 (2H, m), 2.05~1.65 (1H, m), 1.25~1.15 (4H, m), 1.12 (1H, m), 0.75 (1H, m)

Example 18: Preparation of 7-(1-hydroxy-5-azaspiro[2,4]heptane-5-yl)-1-cyclopropyl-6,8-difluoro-1,4-dihyro-4-oxoquinoline-3-carboxylicacid

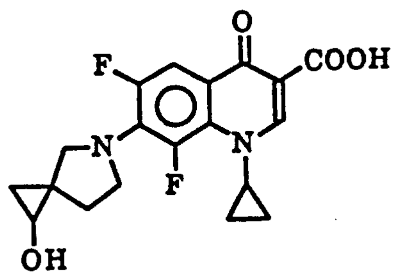

142mg of 1-cyclopropyl-6,7,8-trifluoro-1,4-dihydro-4-oxoquinoline-3-carboxylic acid, 80mg of 1-hydroxy-5-azaspiro[2,4] heptane and 100μℓ of diisopropylethylamine were suspended in 8mℓ of acetonitrile; and the resulting solution was refluxed for 7 hours and left at a room temperature over night. The precipitate produced was filtered, washed with chilled acetonitrile, water and ethylether in turn and dried under a reduced pressure to obtain 132mg of the desired compound(yield: 70%).

m.p. 167~171 °C

$^1$H-NMR (DMSO-$d_6$, δ ppm) : 8.62 (1H, s), 7.70 (1H, d, J = 14.2 Hz), 4.02 (1H, m), 3.80~3.71 (4H, m), 2.32 (1H, m), 1.82 (1H, m), 1.62 (1H, m), 1.12 (4H, m), 0.81 (1H, dd, J = 7.2, 4.5 Hz), 0.42 (1H, t J = 4.5 Hz)

Example 19: Preparation of 7-(1-amino-5-azaspiro[2,4]heptane-5-yl)-1-cyclopropyl-8-methoxy-6-fluoro-1,4-dihydro-4-oxoquinoline-3-carboxylic acid

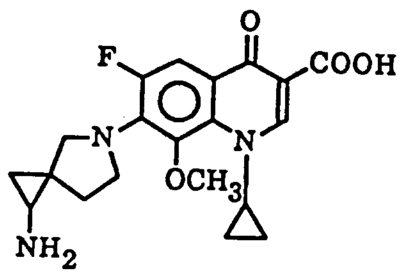

530mg of 1-cyclopropyl-8-methoxy-6,7-difluoro-1,4-dihydro-4-oxoquinoline-3-carboxylic acid, 520mg of 1-amino-5-azaspiro[2,4] heptane hydrobromide and 878μℓ of 1,8-diazabicyclo[5,4,0]undec-7-ene were dissolved in 10mℓ of acetonitrile; and the resulting solution was refluxed for 4 hours and concentrated under a reduced pressure. The resultant was dissolved in 30mℓ of chloroform and washed with water several time. The organic layer was dried over magnesium sulfate and concentrated under a reduced pressure. The resulting material thus obtained was fractionated on silica gel column chromatography(chloroform methanol: water = 15:3:1) to obtain 300mg of the desired compound(yield 50%).

m.p. 183~185 °C

$^1$H-NMR ($CDCl_3$, δ ppm) : 8.71 (1H, s), 7.72 (1H, d, J = 14 Hz), 3.95 (1H, m), 3.76~3.73 (4H, m), 3.63 (3H, s), 2.39 (1H, m), 2.10~1.69 (2H, m), 1.31~0.80 (5H, m), 0.48 (1H, m)

Example 20: Preparation of 7-(1-t-butoxycarbonylamino-5-azaspiro [2,4]heptane-5-yl)-1-cyclopropyl-6,8-difluoro-1,4-dihydro-4-oxoquinoline-3-carboxylic acid

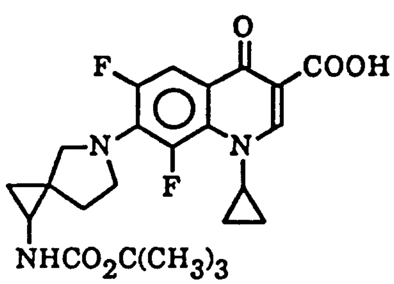

250mg of 1-t-butoxycarbonylamino-5-azaspiro[2,4]heptane, 283mg of 1-cyclopropyl-6,7,8-trifluoro-1,4-dihydro-4-oxoquinoline-3-carboxylic acid and 200mg of 1,8-diazabicyclo[5,4,0]undec-7-ene were dissolved in 10mℓ of acetonitrile; and the resulting solution was refluxed for 3 hours and left at a room temperature over night. The precipitate produced was filtered, washed with acetonitrile and dried under a reduced pressure to obtain 380mg of the desired compound(yield: 80%).

m.p.: 215~219 °C (decomposition)

$^1$H-NMR (DMSO-$d_6$, δ ppm) : 8.57 (1H, s), 7.65 (1H, d, J = 14.2 Hz), 4.05 (1H, m), 3.80~3.68 (4H, m), 2.30 (1H, dd, J = 7.2, 4.8 Hz), 1.85 (1H, m), 1.66 (1H, m), 1.46 (9H, s), 1.12 (4H, m), 0.79 (1H, dd, J = 7.2, 4.5 Hz), 0.41 (1H, t, J = 4.5 Hz)

Example 21: Preparation of 7-(1-amino-5-azaspiro[2,4]heptane-5-yl)-1-cyclopropyl-6,8-difluoro-1,4-dihydro-4-oxoquinoline-3-carboxylic acid hydrochloride

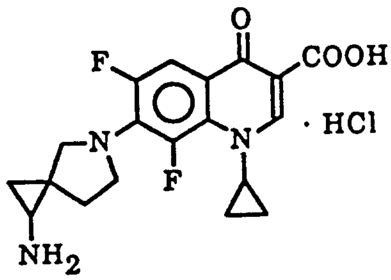

100mg of 7-(1-t-butoxycarbonylamino-5-azaspiro[2,4]heptane-5-yl)-1-cyclopropyl-6,8-difluoro-1,4-dihydro-4-oxoquinoline-3-carboxylic acid prepared in Example 20 was dissolved in 15mℓ of 0.5N hydrochloric acid-methanol solution; and the resulting solution was stirred at room temperature over night and evaporated under a reduced pressure to remove the solvent. 10mℓ of ethylether was added thereto to obtain the powdery material which was filtered and dried under a reduced pressure to obtain quantitatively the desired compound.
m.p.: 159~163 °C (decomposition)

Example 22: Preparation of 7-(1-amino-5-azaspiro[2,4]heptane-5-yl)-1-ethyl-6,8-difluoro-1,4-dihydro-4-oxoquinoline-3-carboxylic acid

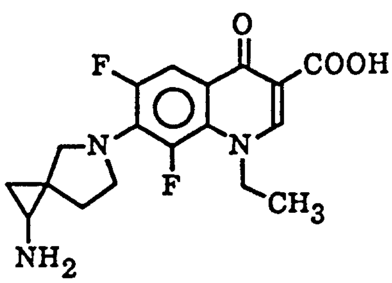

270mg of 1-ethyl-6,7,8-trifluoro-1,4-dihydro-4-oxoquinoline-3-carboxylic acid, 150mg of 1-amino-5-azaspiro[2,4] heptane and 150mg of 1,8-diazabicyclo[5,4,0]undec-7-ene were dissolved in 5mℓ of acetonitrile; and the resulting solution was subjected to the same process as described in Example 1 to obtain 236mg of the desired compound(yield: 65%).
m.p.: 277~180 °C
$^1$H-NMR (DMSO-$d_6$, δ ppm) : 7.88 (1H, d, J = 14.2 Hz, s), 8.75 (1H, s), 4.21 (2H, q, J = 7 Hz), 3.80~3.65 (4H, m), 2.30 (1H, dd, J = 7.2, 4.8 Hz), 1.85 (1H, m), 1.68 (1H, m), 1.45 (3H, t, J = 7 Hz), 0.81 (1H, dd, J = 7.2, 4.5 Hz), 0.42 (1H, t, J = 4.5 Hz)

Example 23: Preparation of 7-(1-amino-5-azaspiro[2,5]octane-5-yl)-1-cyclopropyl-6-fluoro-4-oxo-1,4-dihydro-1,8-naphthyridine-3-carboxylic acid

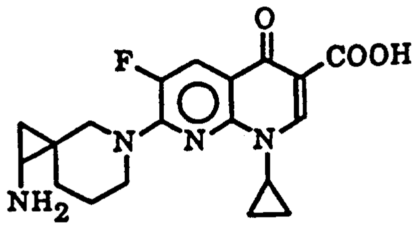

140mg of 1-cyclopropyl-7-chloro-6-fluoro-4-oxo-1,4-dihydro-1,8-naphthyridine-3-carboxylic acid, 120mg of 1-amino-5-azaspiro [2,5]octane hydrochloride and 220mg of 1,8-diazabicyclo[5,4,0]undec-7-enewere dissolved in 5mℓ of acetonitrile; and the resulting solution was subjected to the same process as described in Example 6 to obtain 112mg of the desired compound (yield: 60%).

m.p.: 220~204 °C

$^1$H-NMR (DMSO-$d_6$, $\delta$ ppm) : 8.55 (1H, s), 7.92 (1H, d, J = 12.7 Hz), 4.01 (1H, m), 3.12 (1H, d, J = 12.5 Hz), 2.85 (1H, d, J = 12.5 Hz), 2.33 (1H, dd, J = 7.3, 4.0 Hz), 1.86~1.64 (4H, m), 1.30~1.10 (4H, J = 4 Hz), 1.30~1.15 (4H, m), 0.72 (1H, dd, J = 7.3, 5.2 Hz), 0.35 (1H, t, J = 4.5 Hz)

Example 24: Preparation of 7-(1-amino-5-azaspiro[2,4]heptane-5-yl)-1-t-butyl-6-fluoro-1,4-dihydro-4-oxoquinoline-3-carboxylic acid

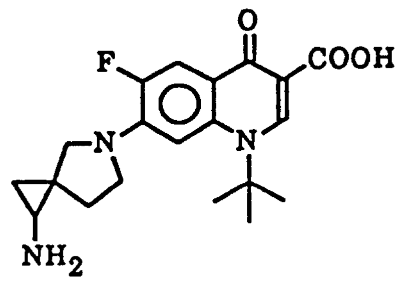

280mg of 1-t-butyl-6,7-difluoro-1,4-dihydro-4-oxoquinoline-3-carboxylic acid, 230mg of 1-amino-5-azaspiro[2,4]heptane hydrobromide and 500mg of 1,8-diasabicyclo[5,4,0]undec-7-ene were dissolved in 10mℓ of acetonitrile; and the resulting solution was subjected to the sane process as described in Example 1 to obtain 243mg of the desired compound(yield: 65%).

m.p.: 159~163 °C

$^1$H-NMR (DMSO-$d_6$, $\delta$ ppm) : 8.50 (1H, s), 7.54 (1H, d, J = 14.2 Hz), 7.05 (1H, J = 7.7 Hz), 3.67~3.51 (4H, m), 2.27 (1H, dd, J = 7.2, 4.8 Hz), 1.99 (1H, m), 1.90 (9H, s), 1.72 (1H, m), 0.81 (1H, dd, J = 7.2, 4.5 Hz), 0.42 (1H, t, J = 4.5 Hz)

Example 25: Preparation of 7-(1-amino-5-azaspiro[2,4]heptane-5-yl)-1-(4-fluorophenyl)-6,8-difluoro-1,4-dihydro-4-oxoquinoline-3-carboxylic acid

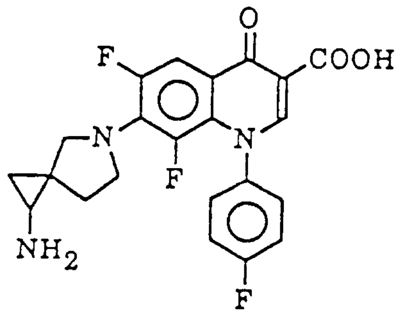

168mg of 1-(4-fluorophenyl)-6,7,8-trifluoro-1,4-dihydro-4-oxoquinoline-3-carboxylic acid, 110mg of 1-amino-5-azaspiro[2,4] heptane hydro bromide and 280mg of 1,8-diazabicyclo[5,4,0]undec-7-ene were dissolved in 8mℓ of acetonitrile; and the resulting solution was subjected to the sane process as described in Example 5 to obtain 120mg of the desired compound(yield: 56%).

m.p.: 175~179 ° C

$^1$H-NMR (DMSO-$d_6$, δ ppm) : 8.58 (1H, s), 8.00 (1H, d, J = 14.2 Hz), 7.46 (2H, m), 7.26 (2H, m), 3.80~3.68 (4H, m), 2.30 (1H, dd, J = 72, 4.8 Hz), 1.85~1.68 (2H, m), 0.79 (1H, dd, J = 7.2, 4.5 Hz), 0.41 (1H, t, J - 4.5 Hz)

Example 26: Preparation of 7-(1-aminomethyl-3-azaspiro[2,4]heptane-5-yl)-1-(2,4-difluorophenyl)-6-fluoro-1,4-dihydro-4-oxoquinoline-3-carboxylic acid

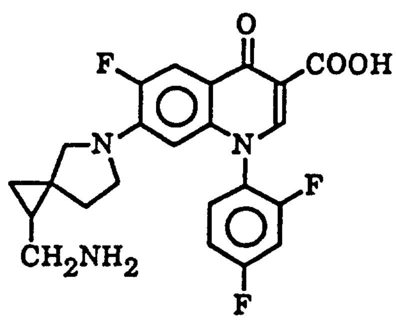

168mg of 1-(2,4-difluorophenyl)-6,7-difluoro-1,4-dihydro-4-oxoquinoline-3-carboxylic acid, 130mg of 1-aminomethyl-5-azaspiro [2,4] heptane formic acid and 150μℓ of diisopropylethylamine were dispersed in 7mℓ of acetonitrile; and the resulting solution was subjected to the same process as described in Example 17 to obtain 122mg of the desired compound(yield: 55%).

m.p.: 181~185 ° C (decomposition)

$^1$H-NMR (DMSO-$d_6$, δ ppm) : 8.85 (1H, s), 8.01 (1H, d, J = 12.2 Hz), 7.46 (1H, m), 7.20 (2H, m), 3.94~3.67 (4H, m), 3.20 (1H, m), 2.90 (1H, m), 2.01~1.85 (2H, m), 1.84 (1H, m), 1.05 (1H, dd, J = 8.5, 5.4 Hz), 0.71 (1H, t, J = 5.4 Hz)

Example 27: Preparation of 7-(1-t-butoxycarbonylamino-5-azaspiro [2,4]heptane-5-yl)-1-cyclopropyl-6,8-difluoro-1,4-dihydro-4-oxoquinoline-3-carboxylic acid ethylester

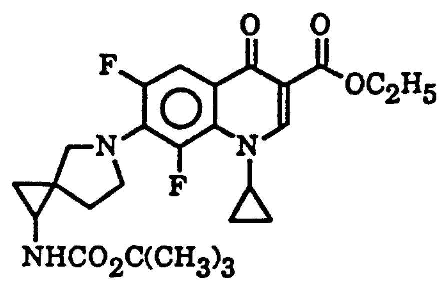

310mg of 1-cyclopropyl-6,7,8-trifluoro-1,4-dihydro-4-oxoquinoline-3-carboxylic acid ethylester, 250mg of 1-t-butoxycarbonylamino-5-azaspiro[2,4]heptane and 500mg of potassium carbonate powder were dispersed in 8mℓ of dimethylformamide; and the resulting solution was stirred at room temperature for 24 hours and evaporated under a reduced pressure to remove the solvent. The residue was then dissolved in 30mℓ of dichloromethane and washed with water; and the organic layer was concentrated. The resultant was crystallized with dichloromethane, ethanol and ethylether to obtain 353mg of the desired compound(yield: 70%).

m.p.: 155~159 °C

$^1$H-NMR (DMSO-$d_6$, δ ppm) : 8.50 (1H, s), 7.62 (1H, d, J = 14.2 Hz), 4.12 (2H, q, J = 7 Hz), 4.01 (1H, m), 3.81~3.65 (4H, m), 2.31 (1H, dd, J = 7.2, 4.8 Hz), 1.82 (1H, m), 1.65 (1H, m), 1.45 (9H, s), 1.25 (3H, t, J = 7 Hz), 1.11 (4H, m), 0.78 (1H, dd, J =7.2, 4.5 Hz), 0.40 (1H, t, J = 4.5 Hz)

Example 28: Preparation of 7-(1-amino-5-azaspiro[2,4]heptane-5yl)-1-cyclopropyl-6,8-difluoro-1,4-dihydro-4-oxoquinoline-3-carboxylic acid ethylester hydrochloride

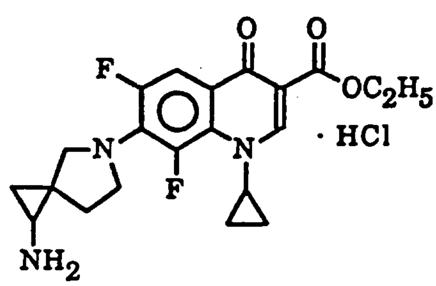

200mg of 7-(1-t-butoxycarbonylamino-5-azaspiro[2,4] heptane-5-yl)-1-cyclopropyl-6,8-difluoro-1,4-dihydro-4-oxoquinoline-3-carboxylic acid ethylester was dissolved in a solvent containing 10mℓ of 0.5N hydrochloric acid-methanol and 2mℓ of dichloromethane; and the resulting solution was stirred at room temperature for 20 hours and evaporated under a reduced pressure to obtain quantitatively the desired compound.

m.p.: 170~174 °C (decomposition)

$^1$H-NMR (DMSO-$d_6$ + $CD_3OD$, δ ppm) : 8.52 (1H, s), 7.65 (1H, d, J = 14.1 Hz), 4.10 (2H, q, J = 7 Hz), 4.00 (1H, m), 3.80~3.66 (4H, m), 2.35 (1H, dd, J = 7.2, 4.7 Hz), 1.83 (1H, m), 1.67 (1H, m), 1.24 (3H, t, J = 7 Hz), 1.15 (4H, m), 0.75 (1H, dd, J = 7.2, 4.5 Hz), 0.41 (1H, t, J = 4.5 Hz)

Example 29: Preparation of 7-(1-amino-5-azaspiro[2,4]heptane-5-yl)-1-cyclopropyl-6,8-difluoro-1,4-dihydro-4-oxoquinoline-3-carboxylic acid

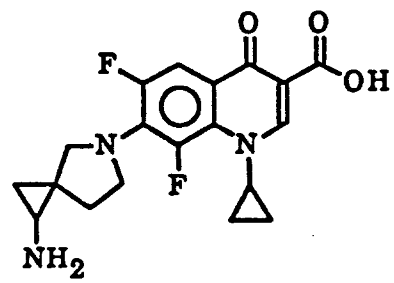

100mg of 7-(1-amino-5-azaspiro[2,4]heptane-5-yl)-1-cyclopropyl-6,8-difluoro-1,4-dihydro-4-oxoquinoline-3-carboxylic acid ethylester prepared in Example 28 was dispersed in a solvent containing 5mℓ of 5% sodium hydroxide and 2mℓ of methanol; and the resulting solution was heated under reflux for 3 hours, cooled to room temperature, neutralized with 2N hydrochloric acid and evaporated under a reduced pressure to remove the solvent. The residue was suspended in 2mℓ of water and filtered. The precipitate was collected and dried under a reduced pressure at 60 ° C to obtain the desired compound(yield: 85%).
m.p.: 217~218 ° C

Example 30: Preparation of 7-(1-acetamino-5-azaspiro[2,4]heptane-5-yl)-1-cyclopropyl-6,8-difluoro-1,4-dihydro-4-oxoquinoline-3-carboxylic acid

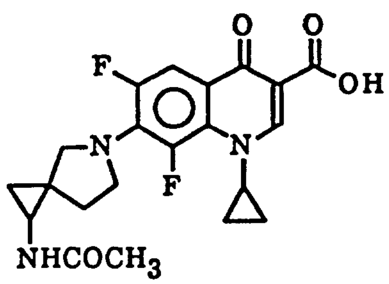

375mg of 7-(1-amino-5-azaspiro[2,4]heptane-5-yl)-1-cyclopropyl-6,8-difluoro-1,4-dihydro-4-oxoquinoline-3-carboxylic acid was dissolved in a solvent containing 5mℓ of pyridine and 4.5mℓ of acetic anhydride; and the resulting solution was stirred at a room temperature over night and evaporated under a reduced pressure to remove the solvent. The residue was dissolved in 2mℓ of dichloromethane and 0.5mℓ of methanol and precipitated by adding 50mℓ of ethylether. The precipitate was filtered and dried under a reduced pressure to obtain 355mg of desired compound(yield: 85%).
m.p.: 190~193 ° C
$^1$H-NMR (DMSO-$d_6$ + $CDCl_3$, δ ppm) : 8.65 (1H, s), 7.65 (1H, d, J = 14 Hz), 4.01 (1H, m), 3.90~3.51 (4H, m), 2.70 (1H, m), 1.90 (3H, s), 1.85~1.67 (2H, m), 1.21~1.05 (5H, m), 0.75 (1H, t, J = 4.5 Hz)

Example 31: Preparation of 7-(1-acetamino-5-azaspiro[2,4]heptane-5-yl )-1-cyclopropyl-6,8-difluoro-1,4-dihydro-4-oxoquinoline-3-carboxylic acid benzyl ester

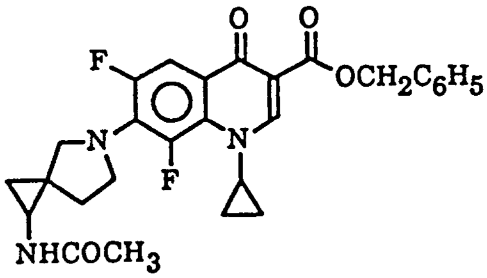

208mg of 7-(1-acetamino-5-azaspiro[2,4]heptane-5-yl)-1-cyclopropyl-6,8-difluoro-1,4-dihydro-4-oxoquinoline-3-carboxylic acid prepared in Example 30 was dissolved in a solvent containing 5mℓ of anhydrous dimethylformamide and 3mℓ of dichloromethane; and the resulting solution was stirred at 50 °C for 20 hours after an addition of 170mg of benzylbromide and 500mg of anhydrous potassium carbonate powder. The reaction mixture was filtered to remove the insoluble material and evaporated under a reduced pressure to remove the solvent. The resulting material thus obtained was fractionated on silica gel column chromatography(chloroform: methanol: water = 15:3:1) to obtain 126mg of the desired compound(yield: 50%).

m.p.: 161~164 °C

$^1$H-NMR (DMSO-$d_6$, δ ppm) : 8.66 (1H, s), 7.67 (1H, d J = 14.1 Hz), 7.42 (5H, m), 5.21 (2H, s), 4.00 (1H, m), 3.91~3.52 (4H, m), 2.65 (1H, m), 1.92 (3H, s), 1.87~1.66 (2H, m), 1.20~1.05 (5H, m), 0.74 (1H, t, J = 4.5 Hz)

Example 32: Preparation of 7-(1-aminoacetylamino-5-azaspiro[2,4] heptane-5-yl)-1-cyclopropyl-6,8-difluoro-1,4-dihydro-4-oxoquinoline-3-carboxylic acid

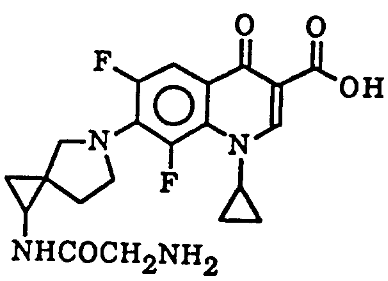

750mg of 7-(1-amino-5-azaspiro[2,4]heptane-5-yl)-1-cyclopropyl-6,8-difluoro-1,4-dihydro-4-oxoquinoline-3-carboxylic acid prepared in Example 1 was dissolved in 7mℓ of pyridine; and the resulting solution was stirred at room temperature for 5 hours after an addition of 600mg of N-(t-butoxycarbonyl)-glycine-4-nitrophenyl ester. The reaction mixture was evaporated under a reduced pressure to remove the solvent. 10mℓ of water was added thereto; and the resulting solution was adjusted to pH 6 with hydrochloric acid. The precipitate thus obtained was filtered, washed with ethanol and ethylether and dried under a reduced pressure to obtain 640mg of 7-(1-t-butoxycarbonyl-aminoacetylamino-5-azaspiro[2,4]heptane-5-yl)-1-cyclopropyl-6,8-difluoro-1,4-dihydro-4-oxoquinoline-3-carboxylic acid(yield: 60%). 600mg of the intermediate was obtained dispersed in 20mℓ of methanol and 2mℓ of concentrated hydrochloric acid and stirred for 5 hours. Methanol and hydrogen chloride were removed under a reduced pressure; and the solution was adjusted to pH 8 with diluted sodium hydroxide. The precipitate formed was filtered, washed with ethanol and ethylether and dried under a reduced pressure to obtain 414mg of the desired compound(yield: 85%).

m.p.: 178~182 °C

Example 33: Preparation of 7-[1-(2-amino-3-methylbutanoyl)-amino-5-azaspiro[2,4]heptane-5-yl]-1-cyclopropyl-8-chloro-1,4-dihydro-4-oxoquinoline-3-carboxylic acid

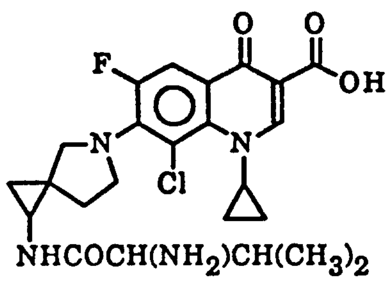

780mg of 7-(1-amino-5-azaspiro[2,4]heptane-5-yl)-1-cyclopropyl-8-chloro-6-fluoro-1,4-dihydro-4-oxoquinoline-3-carboxylic acid and 680mg of N-(t-butoxycarbonyl)-L-valine-4-nitrophenyl ester were dissolved in 15mℓ of pyridine; and the resulting solution was subjected to the same process as described in Example 31 to obtain 768mg of 7-{1-[2-(t-butoxycarbonylamino)-3-methylbutanoyl]-amino-5-azaspiro[2,4]-heptane-5-yl}-1-cyclopropyl-8-chloro-6-fluoro-1,4-dihydro-4-oxoquinoline-3-carboxylic acid(yield: 65%). 750mg of the compound obtained was dispersed in 20mℓ of methanol and 2mℓ of concentrated hydrochloric acid; and the resulting solution was subjected to the same process as described in Example 31 to obtain 500mg of the desired compound(yield: 80%).
m.p.: 200~205 °C(decomposition)

Example 34: Preparation of 7-[1-(N-formyl-N-methyl)-amino-5-azaspiro [2,4]heptane-5-yl]-1-cyclopropyl-8-chloro-6-fluoro-1,4-dihydro-4-oxoquinoline-3-carboxylic acid

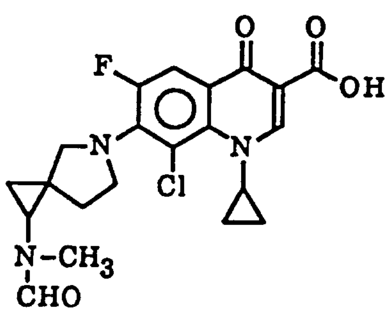

405mg of 7-(1-methylamino-5-azaspiro[2,4]heptane-5-yl)-1-cyclopropyl-8-chloro-6-fluoro-1,4-dihydro-4-oxoquinoline-3-carboxylic acid was dissolved in 15mℓ of pyridine; and the resulting solution was stirred at room temperature for 15 hours after an addition of 1mℓ of acetic formic anhydride. The reaction mixture was evaporated under a reduced pressure to remove the solvent; and 10mℓ of water was added thereto. The resulting solution was adjusted to pH 6 with diluted hydrochloric acid. The precipitate thus formed was filtered, washed with ethanol and ethylether and dried at 60 °C under a reduced pressure to obtain 350mg of the desired compound (yield: 80%).
m.p.: 166~168 °C
$^1$H-NMR ($CDCl_3$, δ ppm) : 9.21 (1H, s), 8.80 (1H, s), 7.90 (1H, d, J = 13.2 Hz), 4.20 (1H, m), 3.90 (2H, m), 3.55 (2H, m), 2.40 (3H, s), 2.11 (1H, m), 1.25~0.97 (4H, m), 0.88 (1H, dd, J = 7.2, 4.5 Hz), 0.56 (1H, t, J = 4.5 Hz)

Example 35: Preparation of 7-(1-amino-5-azaspiro[2,4]heptane-5-yl)-6-fluoro-5-methyl-1-(2,4-difluorophenyl)-1,4-dihydro-4-oxoquinoline-3-carboxylic acid

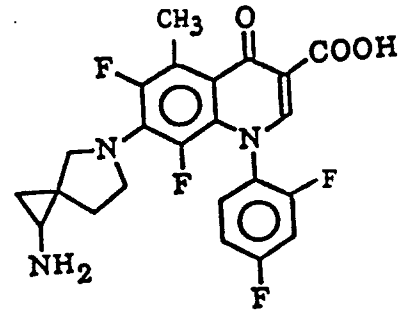

0.7g(2mmol) of 1-(2,4-difluorophenyl)-6,7-difluoro-5-methyl-1,4-dihydro-4-oxoquinoline-3-carboxylic acid, 0.41g(2.2mmol) of 1-amino-5-azaspiro[2,4]heptane hydrochloride and 0.94g(6.2mmol) of 1,8-diazabicyclo-[5,4,0]undec-7-ene were dissolved in 5mℓ of acetonitrile; and the resulting solution was refluxed for 5 hours and cooled to room temperature. The solid produced was filtered, washed with chilled acetonitrile, water and ethylether in turn and dried under a reduced pressure to obtain 0.40g of the desired compound (yield: 45%).

m.p.: 210~212 °C

$^1$H-NMR($CDCl_3$, δ ppm): 0.54(1H, t, J=4.3Hz), 0.87(1H, dd, J=7.2, 5.5Hz), 1.96(1H, m), 2.20(1H, m), 2.46(1H, dd J=7.2, 3.9Hz), 2.71(3H, d, J=2.5Hz), 3.75 (4H, m), 7.08, 1.39-(3H, 2m), 8.25(1H, m), 8.75(1H, s)

Example 36: Preparation of 7-(1-amino-5-azaspiro[2,4]heptane-5-yl)-6-fluoro-5-methyl-1-cyclopropyl-1,4-dihydro-4-oxoquinoline-3-carboxylic acid

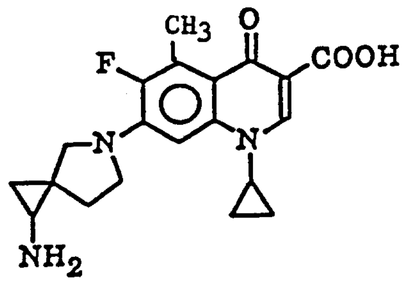

2.3g(8.2mmol) of 1-cyclopropyl-6-fluoro-5-methyl-1,4-dihydro-4-oxoquinoline-3-carboxylic acid, 1.7g-(9.2mmol) of 1-amino-5-azaspiro[2,4]heptane hydrochloride and 4.1g(26.9:mmol) of 1,8-diazabicyclo[5,4,0]-undec-7-ene were dissolved in 20mℓ of acetonitriie; and the resulting solution was refluxed for 3 hours and cooled to room temperature. The solid produced was filtered, washed with chilled acetonitrile, water and ethylether in turn and dried under a reduced pressure to obtain 1.80g of the desired compound (yield: 59.1%).

m.p.: 232~233 °C

$^1$H-NMR($CDCl_3$, δ ppm): 0.55(1H, t, J=4.3Hz), 0.86(1H, dd, J=7.2, 5.5Hz), 1.19(4H, m),1.96(1H, m), 2.21(1H, m), 2.45(1H, dd, J=7.2, 3.9Hz), 2.77(3H, d, J=2.5Hz), 3.75(5H, m), 8.25(1H, m), 8.75(1H, s)

Example 37: Preparation of 7-(1-amino-5-azaspiro[2,4]heptane-5-yl)-6-fluoro-5-methyl-1-cyclopropyl-4-oxo-1,4-dihydro-1,8-naphthyridine-3-carboxylic acid

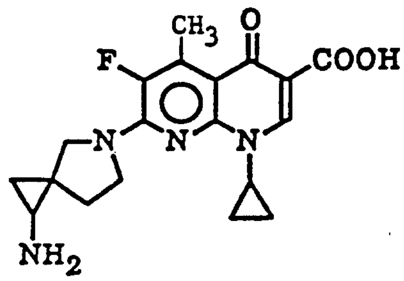

0.89g(3mmol) of 7-chloro-6-fluoro-5-methyl-1-cyclopropyl-4-oxo-1,4-dihydro-1,8-naphthyridine-3-carboxylic acid, 0.61g(3.3mmol) of 1-amino-5-azaspiro[2,4]heptane hydrochloride and 1.46g(9.6mmol) of 1,8-diazabicyclo[5,4,0]undec-7-ene were dissolved in 10mℓ of acetonitrile; and the resulting solution was refluxed for 3 hours and cooled to room temperature. The solid produced was filtered, washed with chilled acetonitrile, water and ethylether in turn and dried under a reduced pressure to obtain 0.57g of the desired compound (yield: 57%).

m.p.: 211~212 °C

$^1$H-NMR($CDCl_3$, δ ppm): 0.54(1H, t, J = 4.3Hz), 0.86(1H, dd, J = 7.2, 5.4Hz), 1.02~1.07(4H, m), 1.96-(1H, m), 2.20 (1H, m), 2.47(1H, dd, J = 7.2, 3.9Hz), 2.75(3H, d, J = 2.5Hz), 3.75(5H, m), 8.43(1H, s)

Example 38: Preparation of 7-(1-amino-5-azaspiro[2,4]heptane-5-yl)-6-fluoro-5-methyl-1-(2,4-difluorophenyl)-4-oxo-1,4-dihydro-1,8-naphthyridine-3-carboxylic acid

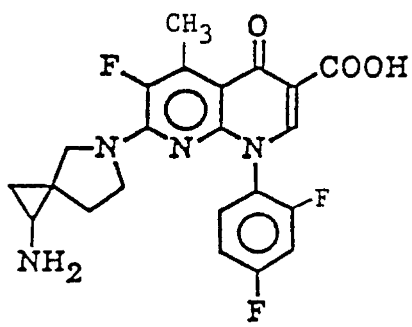

0.92g(2.5mmol) of 7-chloro-6-fluoro-5-methyl-1-(2,4-difluorophenyl)-4-oxo-1,4-dihydro-1,8-naphthyridine)-3-carboxylic acid, 0.51g(2.7mmol) of 1-amino-5-azaspiro[2,4]heptane hydrochloride and 1.2g(7.9mmol) of 1,8-diazabicyclo[5,4,0]undec-7-ene were dissolved in 10mℓ of acetonitrile; and the resulting solution was refluxed for 8 hours and cooled to room temperature. The solid produced was filtered, washed with chilled acetonitrile, water and ethylether in turn and dried under a reduced pressure to obtain 0.49g of the desired compound(yield: 44.1%).

m.p.: 201~202 °C

$^1$H-NMR($CDCl_3$, δ ppm): 0. 54(1H, t, J = 4.4Hz), 0.89(1H, dd, J = 7.2, 5.5Hz), 1.96(1H, m), 2.20(1H, m), 2.46(1H, dd, J = 7.2, 4.0Hz), 2.81(3H, d, J = 2.5Hz), 3.74 (5H, m), 7.08, 7.39-(3H, 2xm), 8.75(1H, s)

Example 39: Preparation of 7-[(1S,3S )-1-amino-5-azaspiro[2,4]heptane-5-yl]-1-cyclopropyl-6,8-difluoro-1,4-dihydro-4oxo-quinoline-3-carboxylic acid

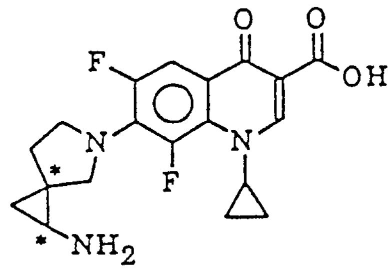

5.3g of 1-cyclopropyl-6,7,8-trifluoro-1,4-dihydro-4-oxo-quinoline-3-carboxylic acid, 3.8g of (1S, 3S)-1-amino-5-azaspiro[2,4] heptane dihydrochloride and 9.4g of 1,8-diazabicyclo[5,4,0]undec-7-ene were dissolved in 30mℓ of acetonitrile; and the resulting solution was refluxed for 5 hours and cooled to room temperature. The solid produced was filtered, washed with chilled acetonitrile, water and ethylether in turn and dried under a reduced pressure to obtain 3.2g of the desired compound(yield: 45.6%).

m.p.: 214 ° C(decompositon)

| | |
|---|---|
| $[\alpha]_D$ : | -66.3 ° (C = 0.347, $CHCl_3$) |
| Ms. m/z (rel. int. %): | 375 ($M^+$, 52), 293 (26), 275 (67), 263(72), 249(27), 40 (100) |
| $^1$H-NMR ($CDCl_3$, $\delta$ ppm) : | 0.53 (1H, t, J = 4.3), 0.86 (1H, dd, J = 7.2, 5.5 Hz), 1.19 (4H, m), 1.96 (1H, m), 2.21 (1H, m), 2.45 (1H, dd, J = 7.2, 3.9 Hz). 3.40 (1H, m), 3.68 (1H, m), 3.88 (2H, m), 7.72 (1H, dd, J = 13.9, 1.62 Hz), 8.69 (1H, s) |

Example 40: Preparation of 7-[(1R, 3R)-1-amino-5-azaspiro[2,4]heptane-5-yl]-1-cyclopropyl-6,8-difluoro-1,4-dihydro-4-oxo-quinoline-3-carboxylic acid

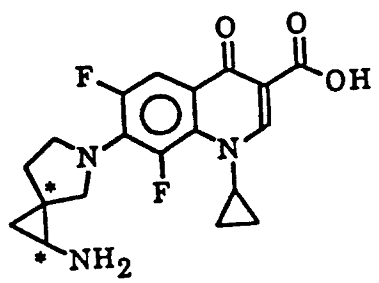

4.2g of 1-cyclopropyl-6,7,8-trifluoro-1,4-dihydro-4-oxoquinoline-3-carboxylic acid, 3.0g of (1R,3R)-1-amino-5-azaspiro [2,4]heptane dihydrochloride and 6.8g of 1,8-diazabicyclo[5,4,0)undec-7-ene were dissolved in 25mℓ of acetonitrile; and the resulting solution was refluxed for 5 hours and cooled to room temperature. The solid produced was filtered, washed with chilled acetonitrile, water and ethylether in turn and dried under a reduced pressure to obtain 3.4g of the desired compound(yield: 61.2%).

m.p.: 205 ° C (decomposition)

| | |
|---|---|
| $[\alpha]_D$ : | -62.0 ° (C = 0.300, $CHCl_3$) |
| Ms. m/z (rel. int. %): | 375 ($M^+$, 72), 356 (26), 293 (38), 275 (91), 263 (100), 249 (33), 82 (27), 41 (25) |
| $^1$H-NMR ($CDCl_3$, $\delta$ ppm) : | 0.54 (1H, t, J = 4.3 Hz), 0.85 (1H, dd, J = 7.2, 5.5 Hz), 1.20 (4H, m), 1.93 (1H, m), 2.19 (1H, m), 2.43 (1H, dd, J = 7.2, 4.0 Hz), 3.41 (1H, m), 3.67 (1H, m), 3.91 (2H, m), 7.73 (1H, dd, J = 13.9, 1.63 Hz), 8.67 (1H, s) |

Example 41: Preparation of 7-[(1S,3R)-1-amino-5-azaspiro[2,4]heptane-5-yl]-1-cyclopropyl-6,8-difluoro-1,4-dihydro-4-oxo-quinoline-3-carboxylic acid

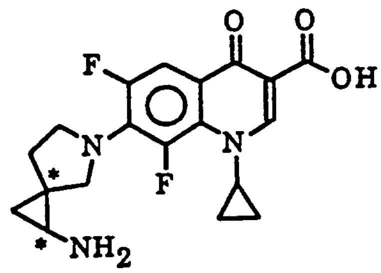

8.5g of 1-cyclopropyl-6,7,8-trifluoro-1,4-dihydro-4-oxoquinoline-3-carboxylic acid, 6.1g of (1S,3R)-1-amino-5-azaspiro [2,4]heptane dihydrochloride and 15.0g of 1,8-diazabicyclo[5,4,0] undec-7-ene were dissolved in 40mℓ of acetonitrile; and the resulting solution was refluxed for 4 hours and cooled to room temperature. The solid produced was filtered, washed with chilled acetonitrile, water and ethylether in turn and dried under a reduced pressure to obtain 7.1g of the desired compound(yield: 63.1%).

m.p.: 185~188°C

| | |
|---|---|
| $[\alpha]_D$ : | +46.4° (C = 0.668, $CHCl_3$) |
| Ms. m/z (rel. int. %): | 375 ($M^+$, 58), 356 (23), 293 (37), 281 (84), 23 (80), 249 (28), 97 (33), 82 (100), 68 (81) |
| $^1H$-NMR ($CDCl_3$, δ ppm) : | 0.49 (1H, t, J = 4.1 Hz), 0.82 (1H, t, J = 5.8 Hz), 1.15 (4H, m), 1.65 (1H, m), 1.39 (1H, m), 2.39 (1H, dd, J = 7.1, 4.1 Hz), 3.80 (4H, m), 7.70 (1H, d, J = 14 Hz), 8.63 (1H, s) |

Example 42: Preparation of 7-[(1R,3R)-1-amino-5-azaspiro[2,4]heptane-5-yl]-1-cyclopropyl-6,8-difluoro-1,4-dihydro-4-oxo-quinoline-3-carboxylic acid

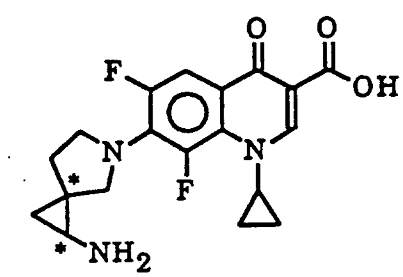

3g of 1-cyclopropyl-6,7,8-trifluoro-1,4-dihydro-4-oxoquinoline-3-carboxylic acid, 2.2g of (1R,3S)-1-amino-5-azaspiro [2,4]heptane dihydrochloride and 5.4g of 1,8-diazabicyclo[5,4,0]undec-7-ene were dissolved in 20mℓ of acetonitrile; and the resulting solution was refluxed for 4 hours and cooled to room temperature. The solid produced was filtered, washed with chilled acetonitrile, water and ethylether in turn and dried under a reduced pressure to obtain 2.1g of the desired compound(yield: 52.8%).

m.p.: 201~203°C

| | |
|---|---|
| $[\alpha]_D$ : | -61.7° (C = 0.321, $CHCl_3$) |
| Ms. m/z (rel. int. %): | 375 $M^+$, 58), 356 (24), 293 (32), 281 (84), 263 (84), 249 (28), 97 (33), 8.2 (100) |
| $^1H$-NMR ($CDCl_3$, δ ppm) : | 0.45 (1H, t, J = 4.0 Hz), 0.77 (1H, dd, J = 7.2,5.4 Hz), 1.10 (4H, m), 1.60 (1H, m), 1.82 (1H, m), 2.32 (1H, dd, J = 7.2,4.0 Hz), 3.74 (4H, m), 7.68 (1H, dd, J = 13.9, 1.9 Hz). 8.49 (1H, s) |

Use Example 1

A capsule formulation was prepared in accordance with the following composition:

| Component | Amount |
|---|---|
| Compound prepared in Example 1 | 100.0mg |
| corn starch | 25.0mg |
| calcium carboxymethyl cellulose | 23.0mg |
| magnesium stearate | 2.0mg |
| total | 150.0mg |

Use Example 2

A solution formulation was prepared in accordance with the following composition:

| Component | Amount |
|---|---|
| Compound prepared in Example 4 | 1 to 10g |
| lactic acid or sodium hydroxide | 0.1 to 2g |
| mannitol | 0.1g |
| deionized water | 87.9 to 98. 8g |
| total | 150g |

The compounds prepared in the Examples were tested as below.

1. In vitro anti-bacterial activity test

In order to demonstrate the superior effectiveness of the quinolone derivatives of the present invention, the minimal inhibitory concentration(MIC) of several compounds synthesized in Examples hereof against the standard strains was determined and compared with ofloxacin and ciprofloxacin. These MIC values were taken by employing two-fold dilution method: that is, two-fold serial dilutions of each of the test compounds were made and dispersed in a Mueller-Hinton agar medium; a standard strain which had the value of $10^7$ CFU/mℓ was inoculated on the medium, which was then incubated at 37°C for 18 hours. The results of the MIC tests are shown in Tables 1 and 2.

Table 1: In vitro anti-bacterial activity test against the standard strains

(MIC : μg/mℓ)

| Strain | Compound prepared in Example1 | Compound prepared in Example2 | Compound prepared in Example3 | Compound prepared in Example4 | Compound prepared in Example5 | Compound prepared in Example6 | Compound prepared in Example7 |
|---|---|---|---|---|---|---|---|
| 1 | 0.195 | 0.195 | 0.098 | 0.098 | 0.391 | 0.391 | 0.391 |
| 2 | 0.098 | 0.098 | 0.025 | 0.049 | 0.391 | 0.195 | 0.195 |
| 3 | 0.195 | 0.098 | 0.391 | 0.098 | 0.195 | 0.195 | 0.391 |
| 4 | 0.025 | 0.025 | 0.025 | 0.025 | 0.098 | 0.049 | 0.025 |
| 5 | 0.025 | 0.025 | 0.007 | 0.025 | 0.098 | 0.049 | 0.025 |
| 6 | 0.025 | 0.025 | 0.004 | 0.013 | 0.098 | 0.049 | 0.025 |
| 7 | 0.004 | 0.004 | <0.002 | 0.007 | 0.025 | <0.002 | 0.007 |
| 8 | 0.195 | 0.195 | 0.195 | 0.098 | 0.781 | 0.391 | 0.391 |
| 9 | 0.049 | 0.049 | 0.025 | 0.049 | 0.098 | 0.098 | 0.098 |
| 10 | 0.013 | 0.025 | 0.007 | 0.025 | 0.049 | 0.025 | 0.025 |
| 11 | 0.013 | 0.025 | 0.025 | 0.025 | 0.098 | 0.025 | 0.098 |
| 12 | 0.781 | 0.781 | 0.781 | 0.781 | 1.563 | 1.563 | 1.563 |
| 13 | 0.391 | 0.781 | 0.781 | 0.781 | 1.563 | 1.563 | 1.563 |
| 14 | 0.391 | 0.781 | 0.781 | 0.781 | 1.563 | 1.563 | 0.781 |
| 15 | 0.195 | 0.098 | 0.195 | 0.195 | 0.391 | 0.195 | 0.391 |
| 16 | 0.013 | 0.013 | 0.004 | 0.025 | 0.049 | 0.025 | 0.025 |
| 17 | 0.004 | 0.013 | <0.002 | <0.002 | 0.004 | 0.391 | 0.007 |
| 18 | 0.013 | 0.025 | 0.004 | 0.025 | 0.049 | 0.025 | 0.098 |
| 19 | 0.013 | 0.013 | 0.004 | 0.025 | 0.049 | 0.007 | 0.025 |
| 20 | 0.004 | 0.007 | <0.002 | 0.007 | 0.025 | <0.002 | 0.013 |

Table 1: (Continued)

(MIC : μg/mℓ)

| Strain | Compound prepared in Example 8 | Compound prepared in Example 9 | Compound prepared in Example 10 | Compound prepared in Example 11 | Compound prepared in Example 12 | Compound prepared in Example 13 | Compound prepared in Example 14 |
|---|---|---|---|---|---|---|---|
| 1 | 0.391 | 0.391 | 0.391 | 0.781 | 0.195 | 0.391 | 3.125 |
| 2 | 0.195 | 0.195 | 0.195 | 0.781 | 0.195 | 0.195 | 1.563 |
| 3 | 0.195 | 0.195 | 0.195 | 0.781 | 0.195 | 0.195 | 1.563 |
| 4 | 0.049 | 0.049 | 0.049 | 0.098 | 0.025 | 0.098 | 0.098 |
| 5 | 0.049 | 0.049 | 0.049 | 0.098 | 0.025 | 0.098 | 0.098 |
| 6 | 0.049 | 0.049 | 0.049 | 0.098 | 0.025 | 0.049 | 0.098 |
| 7 | 0.013 | 0.025 | 0.025 | 0.049 | 0.025 | 0.025 | 0.195 |
| 8 | 0.391 | 0.391 | 0.391 | 1.563 | 0.391 | 0.391 | 6.250 |
| 9 | 0.098 | 0.098 | 0.049 | 0.195 | 0.195 | 0.098 | 0.391 |
| 10 | 0.049 | 0.049 | 0.049 | 0.098 | 0.049 | 0.049 | 0.391 |
| 11 | 0.049 | 0.098 | 0.098 | 0.098 | 0.049 | 0.098 | 0.781 |
| 12 | 1.563 | 3.125 | 1.563 | 3.125 | 3.125 | 1.563 | 12.500 |
| 13 | 1.563 | 1.563 | 1.563 | 3.125 | 3.125 | 1.563 | 6.250 |
| 14 | 1.563 | 1.563 | 1.563 | 3.125 | 1.563 | 1.563 | 12.500 |
| 15 | 0.391 | 0.781 | 0.781 | 0.781 | 0.391 | 0.391 | 1.563 |
| 16 | 0.049 | 0.049 | 0.049 | 0.098 | 0.049 | 0.049 | 0.781 |
| 17 | 0.004 | 0.025 | 0.007 | 0.004 | <0.002 | 0.004 | 0.098 |
| 18 | 0.098 | 0.195 | 0.098 | 0.098 | 0.098 | 0.098 | 1.563 |
| 19 | 0.049 | 0.098 | 0.049 | 0.098 | 0.049 | 0.049 | 0.391 |
| 20 | 0.025 | 0.025 | 0.025 | 0.049 | 0.025 | 0.049 | 0.195 |

Table 1: (Continued)

(MIC : μg/mℓ)

| Strain | Compound prepared in Example 15 | Compound prepared in Example 16 | Compound prepared in Example 19 | Cipro-floxacin | Ofloxacin |
|---|---|---|---|---|---|
| 1 | 0.781 | 0.781 | 0.195 | 3.125 | 3.125 |
| 2 | 0.195 | 0.391 | 0.098 | 0.781 | 1.563 |
| 3 | 0.391 | 0.391 | 0.098 | 0.781 | 0.781 |
| 4 | 0.098 | 0.098 | 0.025 | 0.195 | 0.195 |
| 5 | 0.195 | 0.098 | 0.049 | 0.781 | 0.391 |
| 6 | 0.195 | 0.098 | 0.025 | 0.781 | 0.391 |
| 7 | 0.049 | 0.049 | 0.025 | 0.002 | 0.013 |
| 8 | 0.781 | 1.563 | 0.391 | 0.391 | 0.391 |
| 9 | 0.098 | 0.195 | 0.049 | 0.195 | 0.195 |
| 10 | 0.098 | 0.098 | 0.049 | 0.007 | 0.049 |
| 11 | 0.098 | 0.098 | 0.049 | 0.007 | 0.049 |
| 12 | 1.563 | 6.250 | 1.563 | 0.391 | 1.563 |
| 13 | 1.563 | 3.125 | 1.563 | 0.391 | 1.563 |
| 14 | 1.563 | 3.125 | 1.563 | 0.391 | 1.563 |
| 15 | 0.391 | 0.781 | 0.391 | 0.098 | 0.391 |
| 16 | 0.098 | 0.049 | 0.049 | 0.013 | 0.025 |
| 17 | 0.025 | 0.013 | 0.007 | 0.007 | <0.002 |
| 18 | 0.195 | 0.098 | 0.098 | 0.013 | 0.049 |
| 19 | 0.195 | 0.049 | 0.049 | 0.013 | 0.025 |
| 20 | 0.049 | 0.049 | 0.025 | 0.004 | 0.013 |

1. Streptococcus pyogenes 308 A
2. Streptococcus pyogenes 77 A
3. Streptococcus faecium MD 8b
4. Staphylococcus aureus SG 511
5. Staphylococcus aureus 285
6. Staphylococcus aureus 503
7. Escherichia coli DC 0
8. Escherichia coli DC 0
9. Escherichia coli DC 2
10. Escherichia coli OTEM
11. Escherichia coli 1507 E
12. Pseudomonas aeruginosa 9027
13. Pseudomonas aeruginosa 1592 E
14. Pseudomonas aeruginosa 1771
15. Pseudomonas aeruginosa 1771 M
16. Salmonella typhimurium
17. Klebsiella aerogenes 1082 E
18. Klebsiella aerogenes 1522 E
19. Enterobacter cloacae P 99
20. Enterobacter cloacae 1321 E

Table 2: <u>In vitro</u> anti-bacterial activity test against methicillin resistant Staphylococcus aureus

(MIC : μg/mℓ)

| Strain | Compound prepared in Example1 | Compound prepared in Example2 | Compound prepared in Example3 | Compound prepared in Example4 | Compound prepared in Example5 | Compound prepared in Example6 |
|---|---|---|---|---|---|---|
| 1 | 0.025 | 0.007 | 0.025 | 0.007 | 0.098 | 0.025 |
| 2 | 0.025 | 0.013 | 0.025 | 0.013 | 0.098 | 0.049 |
| 3 | 0.049 | 0.013 | 0.025 | 0.013 | 0.098 | 0.049 |
| 4 | 0.025 | 0.007 | 0.025 | 0.007 | 0.098 | 0.049 |
| 5 | 0.013 | <0.002 | 0.013 | <0.002 | 0.049 | 0.025 |
| 6 | 0.013 | <0.002 | 0.013 | <0.002 | 0.049 | 0.025 |
| 7 | 0.013 | 0.004 | 0.013 | 0.004 | 0.049 | 0.025 |
| 8 | 0.049 | 0.013 | 0.025 | 0.013 | 0.195 | 0.049 |
| 9 | 0.025 | 0.007 | 0.013 | 0.007 | 0.049 | 0.025 |
| 10 | 0.025 | 0.007 | 0.013 | 0.007 | 0.049 | 0.025 |
| 11 | 0.049 | 0.013 | 0.025 | 0.013 | 0.098 | 0.049 |
| 12 | 0.049 | 0.013 | 0.025 | 0.013 | 0.098 | 0.049 |
| 13 | 0.025 | 0.013 | 0.025 | 0.013 | 0.098 | 0.049 |
| 14 | 0.025 | 0.013 | 0.025 | 0.013 | 0.098 | 0.049 |
| 15 | 0.049 | 0.013 | 0.025 | 0.013 | 0.195 | 0.049 |
| 16 | 0.013 | <0.002 | 0.013 | <0.002 | 0.025 | 0.013 |
| 17 | 0.013 | 0.004 | 0.013 | 0.004 | 0.049 | 0.025 |
| 18 | 0.025 | 0.004 | 0.013 | 0.004 | 0.049 | 0.025 |
| 19 | 0.025 | 0.007 | 0.025 | 0.007 | 0.098 | 0.025 |

Table 2: (Continued)

(MIC : μg/mℓ)

| Strain | Compound prepared in Example7 | Compound prepared in Example8 | Compound prepared in Example9 | Ofloxacin | Cipro-floxcain |
|---|---|---|---|---|---|
| 1 | 0.098 | 0.098 | 0.195 | 0.781 | 1.563 |
| 2 | 0.025 | 0.098 | 0.098 | 0.781 | 0.781 |
| 3 | 0.025 | 0.098 | 0.098 | 0.781 | 0.781 |
| 4 | 0.025 | 0.195 | 0.098 | 0.781 | 1.563 |
| 5 | 0.013 | 0.049 | 0.049 | 0.391 | 0.781 |
| 6 | 0.013 | 0.049 | 0.049 | 0.391 | 0.391 |
| 7 | 0.013 | 0.049 | 0.049 | 0.391 | 0.781 |
| 8 | 0.025 | 0.391 | 0.098 | 0.781 | 0.781 |
| 9 | 0.013 | 0.049 | 0.049 | 0.391 | 0.781 |
| 10 | 0.013 | 0.049 | 0.049 | 0.391 | 0.391 |
| 11 | 0.025 | 0.195 | 0.098 | 0.781 | 0.781 |
| 12 | 0.025 | 0.195 | 0.098 | 0.781 | 0.781 |
| 13 | 0.025 | 0.195 | 0.098 | 0.781 | 0.781 |
| 14 | 0.025 | 0.098 | 0.098 | 0.781 | 0.781 |
| 15 | 0.025 | 0.195 | 0.098 | 0.781 | 0.781 |
| 16 | 0.007 | 0.049 | 0.025 | 0.195 | 0.195 |
| 17 | 0.007 | 0.049 | 0.025 | 0.391 | 0.781 |
| 18 | 0.013 | 0.049 | 0.098 | 0.391 | 0.781 |
| 19 | 0.025 | 0.098 | 0.098 | 0.391 | 0.781 |

1. *Staphylococcus aureus* 88 E
2. *Staphylococcus aureus* 121 E
3. *Staphylococcus aureus* 208 E
4. *Staphylococcus aureus* 256 E
5. *Staphylococcus aureus* 690 E
6. *Staphylococcus aureus* 692 E
7. *Staphylococcus aureus* 693 E
8. *Staphylococcus aureus* 694 E
9. *Staphylococcus aureus* 695 E
10. *Staphylococcus aureus* 697 E
11. *Staphylococcus aureus* 701 E
12. *Staphylococcus aureus* 703 E
13. *Staphylococcus aureus* 705 E
14. *Staphylococcus aureus* 706 E
15. *Staphylococcus aureus* 707 E
16. *Staphylococcus aureus* 708 E
17. *Staphylococcus aureus* 711 E
18. *Staphylococcus aureus* 714 E
19. *Staphylococcus aureus* 725 E

2. Preventive effect against the systemic infection

10 fold of the pathogen which leads to 100% lethality were injected intraperitoneally to male and female NMRI mice weighing 18 to 20g. Immediately and after 4 hours, the dose of test compounds which was

determined by two-fold serial dilution method was administered orally or subcutaneously; and on day 7, the effectiveness was evaluated in terms of $ED_{50}$ calculated from the number of survived mice by the probit analysis.

Table 3

| Preventive effect against the systemic infection | | | |
|---|---|---|---|
| Pathogen | Compound | $ED_{50}$: mg/kg | |
| | | Subcutaneous Injection | Oral Administration |
| S. aureus 17740 | Compound prepared in Example 1 | 16.8 | 127.7 |
| | Compound prepared in Example 2 | 97.6 | 49.7 |
| | Ofloxacin | 200.0 | >200.0 |
| | Ciprofloxacin | 132.3 | >200.0 |
| P. mirabilis | Compound prepared in Example 1 | 1.36 | 7.13 |
| | Compound prepared in Example 3 | 0.63 | 2.58 |
| | Ofloxacin | 0.33 | 2.21 |
| | Ciprofloxacin | 0.16 | 0.82 |

3. Acute Toxicity Test

Test compounds were administered to female ICR mice weighing 20 to 25g and on day 14, $LD_{50}$ was calculated from the number of survived mice by the probit analysis.

Table 4

| Result of the Acute Toxicity Test | | |
|---|---|---|
| Compound | Acute Toxicity($LD_{50}$: mg/kg) | |
| | Intraperitoneal Injection | Oral Administration |
| Compound prepared in Example 1 | 605 | 3800 |
| Compound prepared in Example 2 | 580 | 3800 |
| Compound prepared in Example 13 | 620 | 4000 |

As can be seen from the results, the compounds of the present invention possess a broad spectrum of potent antibacterial activities against gram-positive and-negative bacteria, particularly, methicillin resistant Staphylococcus aureus as compared with the known quinolone antibiotics, ciprofloxacin and ofloxacin. The compounds of the present invention also exhibit superior activities to the known quinolone antibiotics in terms of 50% effective dose ($ED_{50}$) on the systemic bacterial infection. Further, the compounds of the present invention have low toxicity sufficient to be proved as drugs, and no effects on the cardiovascular system of the dogs, particularly blood pressure-lowering effect.

Accordingly, the compounds of the present invention will be useful as therapeutical compounds and preservatives of inorganic and organic material.

## Claims

**1.** A quinolone carboxylic acid derivative of formula(I) and pharmaceutically acceptable salts and hydrates thereof:

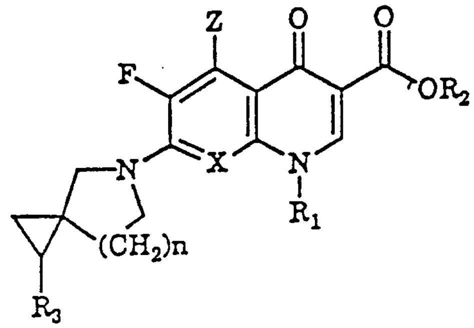

(I)

| | |
|---|---|
| X is | a nitrogen atom or a CH, C-halogen or C-methoxy group; |
| $R_1$ is | a $C_{1-7}$ alkyl group optionally substituted with a halogen atom or a hydroxy radical,a $C_{3-7}$ cycloalkyl group optionally substituted with cis-fluoro or cis-methyl radical, an optionally substituted alkenyl group an aryl group optionally substituted with at least one halogen atom, hydroxy, alkyl, alkoxy, amino or nitro radical or a divalent group of $-CH_2CH_2{}^*CH(CH_3)-$, $-OCH_2{}^*CH(CH_3)$-or $-SCH_2{}^*CH(CH_3)-$ which forms a ring together with the nitrogen atom to which $R_1$ is attached and with X wherein X is C ; |
| $R_2$ is | a hydrogen atom, a carboxy protecting group or a pharmaceutically acceptable metal or organic cation; |
| $R_3$ is | $-CO_2H$ or $-(CH_2)_m-Y$, wherein m is 0 or 1 and Y is a hydroxy group, a $C_{1-4}$ alkoxy group, a $C_{1-4}$ alkoxycarbonyl group (wherein m is 0) or an amino group optionally substituted with one or two $C_{1-4}$ alkyl radicals, with one $C_{1-4}$ alkanoyl radical or with one nitrogen protecting radical metabolizable in vivo which is a formyl group, a $C_{1-4}$ alkoxycarbonyl group, a $C_{1-4}$ alkoxycarbonyl methyl group, a 2 or 3-oxoalkyl group, a 4-methylene-5-methyl-1,3-dioxolene-2-one group or a $RCH(NH_2)CO$ group derived from amino acids wherein R is a hydrogen atom, an optionally substituted $C_1$-$C_4$ alkyl group or a phenyl group, and the carbon atom to which $R_3$ is attached is a chiral carbon atom having the stereochemical configuration of (R), (S) or a mixture thereof; |
| Z is | a hydrogen, chlorine or fluorine atom, a hydroxy group, a methyl group or an amino group optionally substituted with an acetyl or methyl radical and |
| n is | 1 or 2. |

**2.** The compound of claim 1 wherein $R_1$ is a methyl, ethyl, isopropyl, t-butyl, t-pentyl, 2-fluoroethyl, 2-hydroxyethyl, vinyl, isopropenyl, 2-butenyl, cyclopropyl, cyclobutyl, phenyl, 4-fluorophenyl or 2,4-difluorophenyl group; $R_2$ is a hydrogen atom, a methyl, ethyl, n-propyl, t-butyl, cyclohexyl, aryl, acetoxymethyl, pivaloyloxymethyl, methoxymethyl or 4-methylene-5-methyl-1,3-dioxorene-2-one group or a sodium, potassium, silver, ammonium or $C_{1-4}$ tertiary or quarternary alkylammonium cation; and $R_3$ is a hydroxy, hydroxymethyl, amino, aminomethyl, $C_{1-4}$ alkylamino, $C_{1-4}$ alkoxycarbonyl, formylamino or $RCH(NH_2)CONH$-group wherein R is a hydrogen atom or an alkyl group optionally substituted with a methyl, ethyl, propyl, isopropyl, butyl, isobutyl, or phenyl radical.

**3.** The compound of claim 1 which is the salt with hydrochloric, sulfuric, phosphoric, methanesulfonic, acetic, p-toluenesulfonic, formic, propionic, lactic, maleic, malonic, fumaric, tartaric, citric, ascorbic or benzensulfonic acid, and the hydrates thereof.

**4.** A process for preparing a compound of formula(Ia), which comprises reacting a compound of formula(II) with a compound of formula(III) or (IIIa):

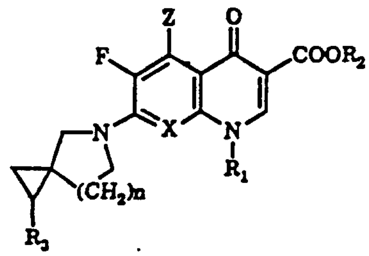

(Ia)

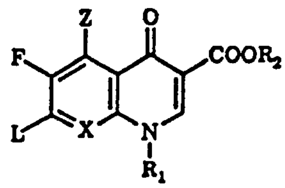

(II)

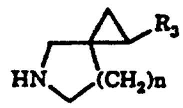

(III)

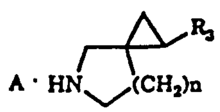

(IIIa)

wherein:

$R_1$, $R_3$, Z, X and n are the same as defined in claim 1;

$R_2$ is a hydrogen atom or a carboxyl protecting group;

L is a leaving group which is a halogen atom or a ethanesulfonyl, benzenesulfonyl onyl or p-toluenesulfonyl group; and

A is a strong acid such as hydrochloric, hydrobromic or sulfuric acid.

**5.** A process for preparing a compound of formula(Ia-1), which comprises acid or alkali hydrolyzing or reducing with hydrogen a compound of formula(Ia-2) wherein $R_2$ is a carboxyl protecting group:

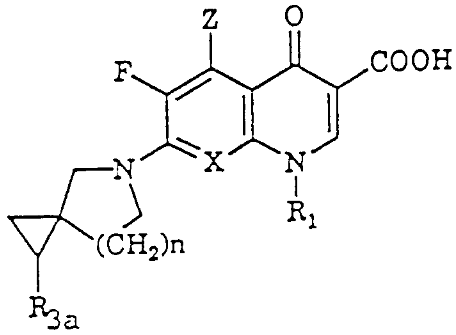

(Ia-1)

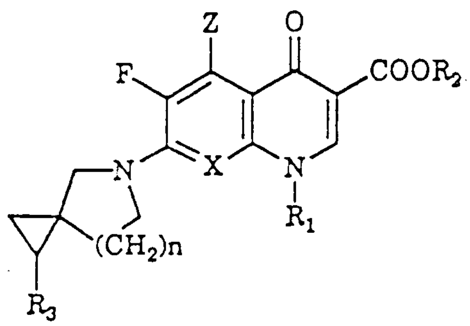

(Ia-2)

wherein:

$R_1$, $R_3$, Z, X and n are the same as defined in claim 1; and

$R_{3a}$ is a group of $-(CH_2)_{m-Y}$ wherein m is 0 or 1 and Y is a hydroxy group or an amino group optionally substituted with one or two $C_1$-$C_4$ alkyl radical or with $RCH(NH_2)CO$ derived from amino acids wherein R is a hydrogen atom, a $C_1$-$C_4$ alkyl group optionally substituted with methyl, ethyl, propyl, isopropyl, butyl, isobutyl, or a phenyl radical.

**6.** A process for preparing a compound of formula(Ia-2) wherein $R_2$ is carboxyl protecting group, which comprises reacting a compound of formula(Ia-1) with a compound of formula $R_2$-Hal(wherein Hal is the same as defined below):

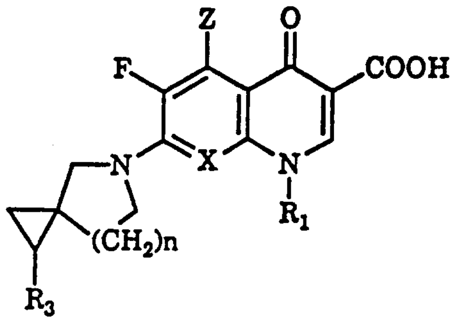

(Ia-1)

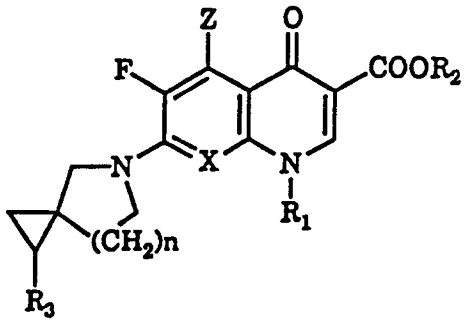

(Ia-2)

wherein:

$R_1$, X, Z and n are the same as defined in claim 1;

$R_3$ is the same as defined in claim 1, excepting an amino or monoalkyl amino group; and

Hal is a chlorine, fluorine or iodine atom.

**7.** A process for preparing a compound of formula(Ia-4) which comprises reacting a compound of formula-(Ia-3) with a compound of formula $R_5$ -W:

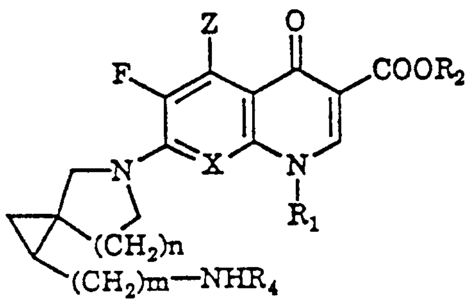

(Ia-3)

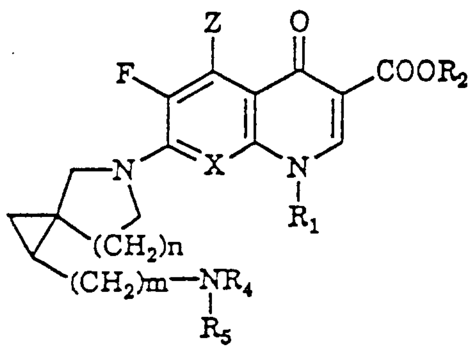

(Ia-4)

wherein:

$R_1$, X, Z and n are the same as defined in claim 1;

$R_2$ is the same as defined in claim 4;

m is 0 or 1;

$R_4$ is a hydrogen atom or a $C_{1-4}$ alkyl group; and

$R_5$ is a nitrogen protecting group metabolizable in vivo which is CN, 4-methylene-5-methyl-1,3-dioxolene-2-one or $(CH_2)_pCO-R_6$ wherein p is 0, 1 or 2 and $R_6$ is a hydrogen atom, an amino group, a straight or branched $C_{1-6}$ alkyl or alkoxy group optionally substituted with a methoxy, ethoxy, fluoro or hydroxy radical, a $C_6$ or $C_{10}$ aryl group optionally substituted with a methyl, methoxy or nitro radical or a straight or branched alkyl group optionally substituted with 1 to 5 substituents selected from a fluorine or chlorine atom, a hydroxy, nitro, amino and carboxy radical or a phenyl group.

**8.** An antibacterial formulation comprising at least one of the compounds of formula(I) as an active component.

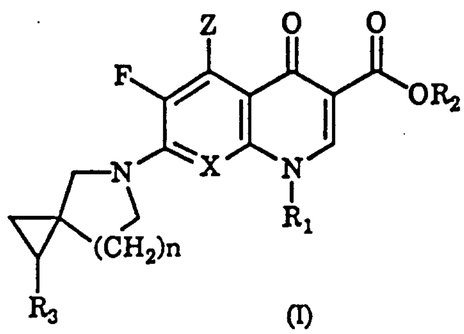

(I)

wherein:

$R_1$, $R_2$, $R_3$, X, Z and n are the same as defined in claim 1.

European Patent Office

# EUROPEAN SEARCH REPORT

Application Number

EP 92 12 1923

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 357 047 (DAIICHI PHAMACEUTICAL CO.)<br>* the whole document *<br>--- | 1-8 | C07D401/04<br>C07D215/56<br>C07D471/04<br>A61K31/47 |
| A | CHEMICAL ABSTRACTS, vol. 113, no. 11, 10 September 1990, Columbus, Ohio, US; abstract no. 97432e, T.P. CULBERTSON ET AL.<br>* abstract *<br>& JOURNAL OF MEDICINAL CHEMISTRY vol. 33, no. 8, 1990, WASHINGTON US pages 2270 - 2275<br>--- | 1-8 | |
| A | EP-A-0 207 420 (DAIICHI SEIYAKU CO. LTD.)<br>* examples 11-16 *<br>--- | 1-8 | |
| A | CHEMICAL ABSTRACTS, vol. 112, no. 7, 12 February 1990, Columbus, Ohio, US; abstract no. 55562w, S.E. HAGEN ET AL.<br>* abstract *<br>& JOURNAL OF MEDICINAL CHEMISTRY vol. 33, no. 2, 1990, WASHINGTON US pages 849 - 854<br>--- | 1-8 | **TECHNICAL FIELDS SEARCHED (Int. Cl.5)** |
| A | DATABASE WPIL Week 9119, Derwent Publications Ltd., London, GB; AN 91-136059 & JP-A-3 072 476 (DAIICHI PHARM. CO. LTD.)<br>* abstract *<br>--- | 1-8 | C07D<br>A61K |
| A | DATABASE WPIL Week 8242, Derwent Publications Ltd., London, GB; AN 82-89588E & JP-A-57 149 286 (DAIICHI SEIYAKU K. K.)<br>* abstract *<br>----- | 1-8 | |

The present search report has been drawn up for all claims

| Place of search | Date of completion of the search | Examiner |
|---|---|---|
| BERLIN | 24 FEBRUARY 1993 | FRELON D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)